# EUROPEAN PATENT SPECIFICATION

(11) **EP 3 849 562 B1**
(45) Date of publication and mention of the grant of the patent: **26.06.2024**
(21) Application number: 19859920.1
(22) Date of filing: 11.09.2019
(51) Int. Cl.: A61K 31/69, A61K 31/546, A61K 31/43, A61K 31/431, A61K 31/496, A61K 9/00, A61K 45/06, A61P 31/04

(54) **COMBINATION COMPOSITIONS COMPRISING A BETA-LACTAMASE INHIBITOR**
KOMBINATIONSZUSAMMENSETZUNGEN MIT EINEM BETA-LACTAMASE-INHIBITOR
COMPOSITIONS SOUS FORME D'ASSOCIATIONS COMPRENANT UN INHIBITEUR DE BÊTA-LACTAMASE

(30) Priority: 12.09.2018 US 201862730289 P
(43) Date of publication of application: 21.07.2021
(73) Proprietor: Venatorx Pharmaceuticals, Inc., Malvern, PA 19355 (US)
(72) Inventor: HAMRICK, Jodie, Malvern, Pennsylvania 19355 (US); BURNS, Christopher J., Malvern, Pennsylvania 19355 (US); PEVEAR, Daniel C., Malvern, Pennsylvania 19355 (US); XERRI, Luigi, Malvern, Pennsylvania 19355 (US); HENKEL, Timothy, Malvern, Pennsylvania 19355 (US); DAIGLE, Denis, Malvern, Pennsylvania 19355 (US)
(74) Representative: Mewburn Ellis LLP
(86) International application number: PCT/US2019/050682
(87) International publication number: WO 2020/056048

(56) References cited:
- WO-A1-2018/165048
- KR-A- 20130 064 004
- US-A1- 2009 156 518
- US-A1- 2014 171 390
- US-A1- 2017 281 639
- Georgiou Panagiota-Christina ET AL: "VNRX-5133, a novel broad-spectrum beta-lactamase inhibitor, enhances the activity of cefepime against resistant Enterobacteriaceae and P. aeruginosa isolates in a neutropenic mouse-thigh infection model", European Congress of Clinical Microbiology and Infectious Diseases, 24 April 2018 (2018-04-24), XP055909612, Retrieved from the Internet: URL:https://www.escmid.org/escmid_publicat ions/escmid_elibrary/material/?mid=64174 [retrieved on 2022-04-06]
- JUSTIN J CHOI ET AL: "Cefiderocol: a novel siderophore cephalosporin", EXPERT OPINION ON INVESTIGATIONAL DRUGS, vol. 27, no. 2, 24 January 2018 (2018-01-24), pages 193-197, XP055634016, UK ISSN: 1354-3784, DOI: 10.1080/13543784.2018.1426745
- P. G. HIGGINS ET AL: "In Vitro Activities of the -Lactamase Inhibitors Clavulanic Acid, Sulbactam, and Tazobactam Alone or in Combination with -Lactams against Epidemiologically Characterized Multidrug-Resistant Acinetobacter baumannii Strains", ANTIMICROBIAL AGENTS AND CHEMOTHERAPY, vol. 48, no. 5, 22 April 2004 (2004-04-22) , pages 1586-1592, XP055023196, ISSN: 0066-4804, DOI: 10.1128/AAC.48.5.1586-1592.2004
- Takayuki Katsube, Roger Echols, Juan Camilo Arjona Ferreira, Heidi K. Krenz, Jolene Kay Berg, Christopher Galloway: "Cefiderocol, a Siderophore Cephalosporin for Gram Negative Bacterial Infections: Pharmacokinetics and Safety in Subjects With Renal Impairment", JOURNAL OF CLINICAL PHARMACOLOGY., LIPPINCOTT CO, HAGERSTOWN, MD, US, vol. 57, no. 5, 1 May 2017 (2017-05-01), pages 584-591, XP055679359, US ISSN: 0091-2700, DOI: 10.1002/jcph.841
- ITO-HORIYAMA TSUKASA ET AL: "Stability of Novel Siderophore Cephalosporin S-649266 against Clinically Relevant Carbapenemases.", July 2016 (2016-07), ANTIMICROBIAL AGENTS AND CHEMOTHERAPY 07 2016, VOL. 60, NR. 7, PAGE(S) 4384 - 4386 ISSN: 1098-6596

## Description

### FIELD OF INVENTION

The present invention relates to combination compositions comprising a beta-lactamase inhibitor and their use for treatment of bacterial infections.

### BACKGROUND OF THE INVENTION

Antibiotics are the most effective drugs for curing bacteria-infectious diseases clinically. They have a wide market due to their advantages of good antibacterial effect with limited side effects. Among them, the beta-lactam class of antibiotics (for example, penicillins, cephalosporins, and carbapenems) are widely used because they have a strong bactericidal effect and low toxicity.

To counter the efficacy of the various beta-lactams, bacteria have evolved to produce variants of beta-lactam deactivating enzymes called beta-lactamases, and in the ability to share this tool inter- and intra-species. These beta-lactamases are categorized as "serine" or "metallo" based, respectively, on presence of a key serine or zinc in the enzyme active site. The rapid spread of this mechanism of bacterial resistance can severely limit beta-lactam treatment options in the hospital and in the community.
Georgiou et al., European Congress of Clinical Microbiology and Infectious Diseases, 24 April 2018 describes β-lactamase inhibitor VNRX-5133 ((R)-3-(2-(trans-4-(2-aminoethylamino)cyclohexyl) acetamido)-2-hydroxy-3,4-dihydro-2H-benzo[e][1,2]oxaborinine-8-carboxylic acid) and its use with cefepime against highly resistant gram-negative bacteria.
Choi et al, Expert Opin. Investig. Drugs, 24 January 2018, Vol. 27, No. 2, pp. 193-197 describes cefiderocol as a siderophore cephalosporin and its antimicrobial activity against a variety of MDR-bacteria.
Higgins et al., Antimicrobial Agents and Chemotherapy, 2004, Vol. 48, No. 5, pp. 1586-1592 describes in vitro activities of β-lactamase inhibitors clavulanic acid, sulbactam, and tazobactam alone and in combination with β-lactams and their activities against multidrug-resistant *Acinetobacter baumannii .*
WO 2018/165048 A1 (Venatorx Pharmaceuticals, Inc.; 13 September 2018) describes certain solid forms and combination compositions comprising the beta-lactamase inhibitor (R)-3-(2-(trans-4-(2-aminoethylamino)cyclohexyl)acetamido)-2-hydroxy-3,4-dihydro-2H-benzo[e][1,2]oxaborinine-8-carboxylic acid.
US 2014/0171390 A1 (Burns et al.; 19 June 2014) describes certain compounds that act as beta-lactamase inhibitors.
Katsube et al., J. Clin. Pharmaco., 2017, Vol. 57, No. 5, pp 584-591 describes cefiderocol as a siderophore cephalosporin antibiotic.

### SUMMARY OF THE INVENTION

The present invention is a pharmaceutical composition for use in a method of treating a bacterial infection, wherein the composition comprises:
(i) (R)-3-(2-(trans-4-(2-aminoethylamino)cyclohexyl)acetamido)-2-hydroxy-3,4-dihydro-2H-benzo[e][1,2]oxaborinine-8-carboxylicacid: 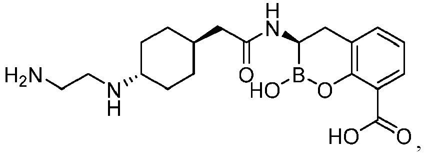 or a pharmaceutically acceptable salt, a solvate, or a pharmaceutically acceptable salt and solvate thereof; and
(ii) a siderophore antibiotic;
   wherein the siderophore antibiotic is cefiderocol.

In some embodiments, the bacterial infection is a resistant bacterial infection. In some embodiments, the bacterial infection is caused by a gram-negative bacterium. In some embodiments, the bacterial infection is caused by *Acinetobacter spp.i,* Enterobacteriaceae, or *Pseudomonas spp.,* or *Stenotrophomonas maltophilia, or Burkholderia spp.* In some embodiments, the pharmaceutical composition is formulated as a liquid suitable for injection. In some embodiments, the pharmaceutical composition is administered as an infusion. In some embodiments, the pharmaceutical composition is administered over a period of about 1 hour to about 4 hours. In some embodiments, the pharmaceutical composition is administered over a period of about 1 hour. In some embodiments, the pharmaceutical composition is administered over a period of about 2 hours. In some embodiments, the pharmaceutical composition is administered over a period of about 3 hours. In some embodiments, the pharmaceutical composition is administered over a period of about 4 hours. In some embodiments, the pharmaceutical composition is administered every about 4 hours to every about 12 hours. In some embodiments, the pharmaceutical composition is administered every about 12 hours. In some embodiments, the pharmaceutical composition is administered every about 8 hours. In some embodiments, the pharmaceutical composition is administered every about 6 hours. In some embodiments, between about 0.125 g and about 1 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 0.125 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 0.25 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 0.5 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 0.75 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 1 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered. In some embodiments, about 2 g of cefiderocol is administered. In some embodiments, the method further comprises administering an additional β-lactam antibiotic. In some embodiments, the additional β-lactam antibiotic is a penicillin, a penem, a carbapenem, a cephalosporin, a cephamycin, a monobactam, or any combinations thereof. In some embodiments, the additional β-lactam antibiotic is amoxicillin, ampicillin, azidocillin, azlocillin, bacampicillin, benzathine benzylpenicillin, benzathine phenoxymethylpenicillin, benzylpenicillin (G), carbenicillin, carindacillin, clometocillin, cloxacillin, dicloxacillin, epicillin, flucloxacillin, hetacillin, mecillinam, metampicillin, meticillin, mezlocillin, nafcillin, oxacillin, penamecillin, pheneticillin, phenoxymethylpenicillin (V), piperacillin, pivampicillin, pivmecillinam, procaine benzylpenicillin, propicillin, sulbenicillin, talampicillin, temocillin, ticarcillin, faropenem, biapenem, ertapenem, doripenem, imipenem, meropenem, panipenem, cefacetrile, cefaclor, cefadroxil, cefalexin, cefaloglycin, cefalonium, cefaloridine, cefalotin, cefamandole, cefapirin, cefatrizine, cefazaflur, cefazedone, cefazolin, cefbuperazone, cefcapene, cefdaloxime, cefdinir, cefditoren, cefepime, cefetamet, cefixime, cefmenoxime, cefmetazole, cefminox, cefodizime, cefonicid, cefoperazone, ceforanide, cefotaxime, cefotetan, cefotiam, cefovecin, cefoxitin, cefozopran, cefpimizole, cefpiramide, cefpirome, cefpodoxime, cefprozil, cefquinome, cefquinome, cefradine, cefroxadine, cefsulodin, ceftaroline fosamil, ceftazidime, cefteram, ceftezole, ceftibuten, ceftiofur, ceftiolene, ceftizoxime, ceftobiprole, ceftriaxone, cefuroxime, cefuzonam, flomoxef, latamoxef, loracarbef, aztreonam, carumonam, nocardicin A, tigemonam, or any combinations thereof. In some embodiments, the additional β-lactam antibiotic is cefepime. In some embodiments, between about 1 g and about 2 g of cefepime is administered. In some embodiments, about 1 g of cefepime is administered. In some embodiments, about 2 g of cefepime is administered.

### BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:
**FIG. 1** shows the efficacy of Human Simulated Regimen of Cefepime combined with Compound 1 at Various Dose Ratios in the Neutropenic Thigh Infection Model with Cefepime-Resistant Clinical Isolates of Enterobacteriaceae and *P. aeruginosa* (n=30).
**FIG. 2** shows the efficacy of Cefepime/Compound 1 at a Human Simulated Regimen of 2 g:0.5 g q8h in the Neutropenic Thigh Infection Model with Cefepime-Resistant Clinical Isolates of Enterobacteriaceae and *P. aeruginosa* (n=30).

### DETAILED DESCRIPTION OF THE INVENTION

Any references herein to methods of treatment refer to the compounds, pharmaceutical compositions, and medicaments of the present invention for use in a such methods.

### Definitions

In the following description, certain specific details are set forth in order to provide a thorough understanding of various embodiments. However, one skilled in the art will understand that the invention may be practiced without these details. In other instances, well-known structures have not been shown or described in detail to avoid unnecessarily obscuring descriptions of the embodiments. Unless the context requires otherwise, throughout the specification and claims which follow, the word "comprise" and variations thereof, such as, "comprises" and "comprising" are to be construed in an open, inclusive sense, that is, as "including, but not limited to." Further, headings provided herein are for convenience only and do not interpret the scope or meaning of the claimed invention.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Also, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The term "antibiotic" refers to a compound or composition which decreases the viability of a microorganism, or which inhibits the growth or proliferation of a microorganism. The phrase "inhibits the growth or proliferation" means increasing the generation time (i.e., the time required for the bacterial cell to divide or for the population to double) by at least about 2-fold. Preferred antibiotics are those which can increase the generation time by at least about 10-fold or more (e.g., at least about 100-fold or even indefinitely, as in total cell death). As used in this disclosure, an antibiotic is further intended to include an antimicrobial, bacteriostatic, or bactericidal agent. Examples of antibiotics suitable for use with respect to the present invention include penicillins, cephalosporins and carbapenems.

The term "β-lactam antibiotic" refers to a compound with antibiotic properties that contains a β-lactam functionality. Non-limiting examples of β-lactam antibiotics useful with respect to the invention include penicillins, cephalosporins, penems, carbapenems, and monobactams.

The term "β-lactamase" denotes a protein capable of inactivating a β-lactam antibiotic. The β-lactamase can be an enzyme which catalyzes the hydrolysis of the β-lactam ring of a β-lactam antibiotic. Of particular interest herein are microbial β-lactamases. The β-lactamase may be, for example, a serine β-lactamase or a metallo- β -lactamase. β-Lactamases of interest include those disclosed in an ongoing website that monitors beta-lactamase nomenclature (www.lahey.org) and in Bush, K. and G. A. Jacoby. 2010. An updated functional classification of β -lactamases. Antimicrob. Agents Chemother. 54:969-976. β-Lactamases of particular interest herein include β -lactamases found in bacteria such as class A β -lactamases including the SHV, CTX-M and KPC subclasses, class B β -lactamases such as VIM, class C β -lactamases (both chromosomal and plasmid-mediated), and class D β-lactamases. The term "β-lactamase inhibitor" refers to a compound which is capable of inhibiting β-lactamase activity. Inhibiting β-lactamase activity means inhibiting the activity of a class A, B, C, or D β-lactamase. For antimicrobial applications, inhibition at a 50% inhibitory concentration is preferably achieved at or below about 100 micrograms/mL, or at or below about 50 micrograms/mL, or at or below about 25 micrograms/mL. The terms "class A", "class B", "class C", and "class D" β-lactamases are understood by those skilled in the art and are described in Bush, K. and G. A. Jacoby. 2010. An updated functional classification of β -lactamases. Antimicrob. Agents Chemother. 54:969-976.

### Compound 1

As used herein, (R)-3-(2-(trans-4-(2-aminoethylamino)cyclohexyl)acetamido)-2-hydroxy-3,4-dihydro-2H-benzo[e][1,2]oxaborinine-8-carboxylic acid is as shown in the structure below: 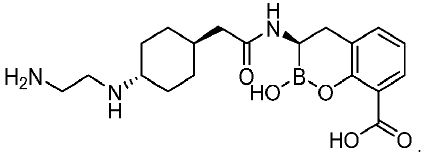 In some embodiments, (R)-3-(2-(trans-4-(2-aminoethylamino)cyclohexyl)acetamido)-2-hydroxy-3,4-dihydro-2H-benzo[e][1,2]oxaborinine-8-carboxylic acid is also referred to as Compound 1. In some embodiments, Compound 1 exists in an equilibrium between the "closed" cyclic form (as shown above) and the "open" acyclic form: 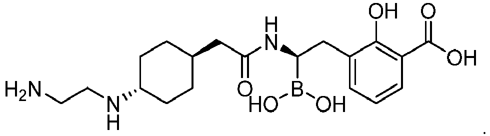 In some embodiments, Compound 1 associates into intramolecular dimers, trimers, and any combinations thereof. In some embodiments, Compound 1 is in the form of a pharmaceutically acceptable salt. In some embodiments, the pharmaceutically acceptable salt is a hydrochloride salt. In some embodiments, the pharmaceutically acceptable salt is a dihydrochloride salt. In some embodiments, Compound 1 is in the form of a pharmaceutically acceptable solvate. Solvates contain either stoichiometric or non-stoichiometric amounts of a solvent, and, in some embodiments, are formed during the process of crystallization with pharmaceutically acceptable solvents such as water or organic solvents. In some embodiments, the solvent is not covalently bound to Compound 1. In some embodiments, the solvent is covalently bound to Compound 1. Hydrates are formed when the solvent is water, or alcoholates are formed when the solvent is alcohol. In general, the solvated forms are considered equivalent to the unsolvated forms for the purposes of the compounds and methods provided herein. In some embodiments, the pharmaceutically acceptable solvate is a monohydrate solvate.

### Combination of Compound 1 with a Siderophore Antibiotic

Disclosed herein is a method of treating a bacterial infection comprising administering a pharmaceutical composition comprising:
(i) ((R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof, 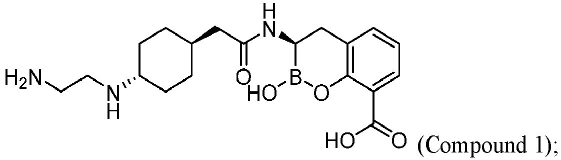 and
(ii) a siderophore antibiotic;
wherein the siderophore antibiotic is cefiderocol.

As used herein a "siderophore antibiotic" refers to an antibiotic that binds to ferric iron and is actively transported into bacterial cells through the outer membrane via the bacterial iron transporters which function to incorporate this essential nutrient for bacteria.

In some embodiments, the bacterial infection is a resistant bacterial infection. In some embodiments, the bacterial infection is caused by a gram-negative bacterium. In some embodiments, the bacterial infection is caused by *Acinetobacter spp* Enterobacteriaceae, or *Pseudomonas spp.,* or *Stenotrophomonas maltophilia, or Burkholderia spp.*

In some embodiments, cefiderocol is administered, by a route and in an amount commonly used therefore, contemporaneously or sequentially with Compound 1, a pharmaceutically acceptable salt, a solvate, or a pharmaceutically acceptable salt and solvate thereof. In some embodiments, Compound 1, a pharmaceutically acceptable salt, a solvate, or a pharmaceutically acceptable salt and solvate thereof, and cefiderocol are administered contemporaneously. In some embodiments, Compound 1, a pharmaceutically acceptable salt, a solvate, or a pharmaceutically acceptable salt and solvate thereof, and cefiderocol are administered sequentially. In some embodiments, Compound 1, a pharmaceutically acceptable salt, a solvate, or a pharmaceutically acceptable salt and solvate thereof, and cefiderocol are administered on different overlapping schedules.

In some embodiments, the pharmaceutical composition is formulated in a conventional manner using one or more pharmaceutically acceptable inactive ingredients that facilitate processing of the active compounds into preparations that can be used pharmaceutically. Proper formulation is dependent upon the route of administration chosen. A summary of pharmaceutical compositions described herein can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

In some embodiments, the pharmaceutical composition comprises at least one pharmaceutically acceptable inactive ingredient. In other embodiments, the pharmaceutical composition includes carriers, adjuvants, preserving, stabilizing, wetting or emulsifying agents, solution promoters, salts for regulating the osmotic pressure, and/or buffers.

In some embodiments, the pharmaceutical composition comprises other chemical components (i.e. pharmaceutically acceptable inactive ingredients), such as carriers, excipients, binders, filling agents, suspending agents, flavoring agents, sweetening agents, disintegrating agents, dispersing agents, surfactants, lubricants, colorants, diluents, solubilizers, moistening agents, plasticizers, stabilizers, penetration enhancers, wetting agents, anti-foaming agents, antioxidants, preservatives, or one or more combination thereof.

The pharmaceutical formulations described herein are administered to a subject by appropriate administration routes, including but not limited to, oral, parenteral (e.g., intravenous, subcutaneous, intramuscular), intranasal, buccal, topical, rectal, or transdermal administration routes. The pharmaceutical formulations described herein include, but are not limited to, aqueous liquid dispersions, liquids, gels, syrups, elixirs, slurries, suspensions, self-emulsifying dispersions, solid solutions, liposomal dispersions, aerosols, solid oral dosage forms, powders, immediate release formulations, controlled release formulations, fast melt formulations, tablets, capsules, pills, powders, dragees, effervescent formulations, lyophilized formulations, delayed release formulations, extended release formulations, pulsatile release formulations, multiparticulate formulations, and mixed immediate and controlled release formulations.

In some embodiments, the pharmaceutical composition is formulated as a liquid suitable for injection.

In some embodiments, the pharmaceutical composition is administered as an infusion. In some embodiments, the pharmaceutical composition is administered as a continuous infusion.

In some embodiments, the pharmaceutical composition is administered over a period of about 1 hour to about 4 hours. In some embodiments, the pharmaceutical composition is administered over a period of about 1 hour. In some embodiments, the pharmaceutical composition is administered over a period of about 2 hours. In some embodiments, the pharmaceutical composition is administered over a period of about 3 hours. In some embodiments, the pharmaceutical composition is administered over a period of about 4 hours.

In some embodiments, the pharmaceutical composition is administered every about 4 hours to every about 12 hours.

In some embodiments, the pharmaceutical composition is administered every about 12 hours. In some embodiments, the pharmaceutical composition is administered every about 10 hours. In some embodiments, the pharmaceutical composition is administered every about 8 hours. In some embodiments, the pharmaceutical composition is administered every about 6 hours. In some embodiments, the pharmaceutical composition is administered every about 4 hours.

In some embodiments, between about 0.125 g and about 1 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, about 0.125 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, about 0.25 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, about 0.5 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, about 0.75 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, about 1 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof is administered.

In some embodiments, between about 0.5 g and about 3 g of cefiderocol is administered. In some embodiments, about 0.5 g of cefiderocol is administered. In some embodiments, about 1 g of cefiderocol is administered. In some embodiments, about 1.5 g of cefiderocol is administered.

In some embodiments, about 2 g of cefiderocol is administered. In some embodiments, about 2.5 g of cefiderocol is administered. In some embodiments, about 3 g of cefiderocol is administered.

In some embodiments, the methods described herein further comprise administering an additional antibiotic. In some embodiments, the methods described herein further comprise administering an additional β-lactam antibiotic.

In some embodiments, the additional β-lactam antibiotic is a penicillin, a penem, a carbapenem, a cephalosporin, a cephamycin, a monobactam, or any combinations thereof.

In some embodiments, the additional β-lactam antibiotic is amoxicillin, ampicillin, azidocillin, azlocillin, bacampicillin, benzathine benzylpenicillin, benzathine phenoxymethylpenicillin, benzylpenicillin (G), carbenicillin, carindacillin, clometocillin, cloxacillin, dicloxacillin, epicillin, flucloxacillin, hetacillin, mecillinam, metampicillin, meticillin, mezlocillin, nafcillin, oxacillin, penamecillin, pheneticillin, phenoxymethylpenicillin (V), piperacillin, pivampicillin, pivmecillinam, procaine benzylpenicillin, propicillin, sulbenicillin, talampicillin, temocillin, ticarcillin, faropenem, biapenem, ertapenem, doripenem, imipenem, meropenem, panipenem, cefacetrile, cefaclor, cefadroxil, cefalexin, cefaloglycin, cefalonium, cefaloridine, cefalotin, cefamandole, cefapirin, cefatrizine, cefazaflur, cefazedone, cefazolin, cefbuperazone, cefcapene, cefdaloxime, cefdinir, cefditoren, cefepime, cefetamet, cefixime, cefmenoxime, cefmetazole, cefminox, cefodizime, cefonicid, cefoperazone, ceforanide, cefotaxime, cefotetan, cefotiam, cefovecin, cefoxitin, cefozopran, cefpimizole, cefpiramide, cefpirome, cefpodoxime, cefprozil, cefquinome, cefquinome, cefradine, cefroxadine, cefsulodin, ceftaroline fosamil, ceftazidime, cefteram, ceftezole, ceftibuten, ceftiofur, ceftiolene, ceftizoxime, ceftobiprole, ceftriaxone, cefuroxime, cefuzonam, flomoxef, latamoxef, loracarbef, aztreonam, carumonam, nocardicin A, tigemonam, or any combinations thereof.

In some embodiments, the additional β-lactam antibiotic is cefepime.

In some embodiments, between about 1 g and about 2 g of cefepime is administered. In some embodiments, about 1 g of cefepime is administered. In some embodiments, about 2 g of cefepime is administered.

Disclosed herein is a method of treating a bacterial infection comprising administering a pharmaceutical composition comprising:
(i) ((R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof, 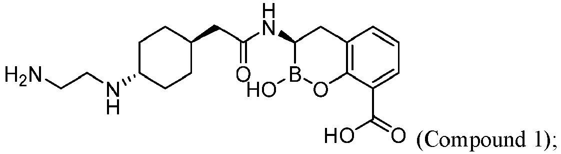 and
(ii) cefiderocol.

In some embodiments, about 2 g cefiderocol and about 0.5 g of Compound 1, a pharmaceutically acceptable salt, a solvate, or a pharmaceutically acceptable salt and solvate thereof, is administered. In some embodiments, about 2 g cefiderocol and about 0.25 g of Compound 1, a pharmaceutically acceptable salt, a solvate, or a pharmaceutically acceptable salt and solvate thereof, is administered. In some embodiments, about 2 g cefiderocol and about 0.125 g of Compound 1, a pharmaceutically acceptable salt, a solvate, or a pharmaceutically acceptable salt and solvate thereof, is administered.

Disclosed herein is a method of treating a bacterial infection comprising administering a pharmaceutical composition comprising:
(i) ((R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof, 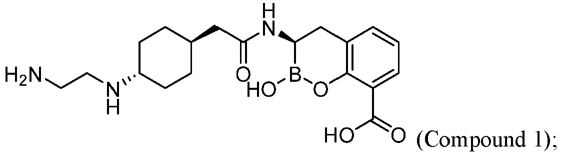
(ii) cefiderocol; and
(iii) cefepime.

In some embodiments, about 2 g cefiderocol, about 2 g of cefepime, and about 0.5 g of Compound 1, a pharmaceutically acceptable salt, a solvate, or a pharmaceutically acceptable salt and solvate thereof, is administered. In some embodiments, about 2 g cefiderocol, about 2 g of cefepime, and about 0.25 g of Compound 1, a pharmaceutically acceptable salt, a solvate, or a pharmaceutically acceptable salt and solvate thereof, is administered. In some embodiments, about 2 g cefiderocol, about 2 g of cefepime, and about 0.125 g of Compound 1, a pharmaceutically acceptable salt, a solvate, or a pharmaceutically acceptable salt and solvate thereof, is administered.

### Methods

Again, any references herein to methods of treatment refer to the compounds, pharmaceutical compositions, and medicaments of the present invention for use in a such methods.

The present disclosure provides methods for inhibiting bacterial growth, by, e.g., reducing bacterial resistance to a β-lactam antibiotic, such methods comprising contacting a bacterial cell culture, or a bacterially infected cell culture, tissue, or organism, with a pharmaceutical composition described herein. Preferably, the bacteria to be inhibited by administration of a pharmaceutical composition described herein are bacteria that are resistant to beta-lactam antibiotics. The term "resistant" is well-understood by those of ordinary skill in the art (see, e g Payne et al., Antimicrobial Agents and Chemotherapy 38 767-772 (1994), Hanaki et al., Antimicrobial Agents and Chemotherapy 30 1120-1126 (1995)).

These methods are useful for inhibiting bacterial growth in a variety of contexts. In certain embodiments, a pharmaceutical composition described herein is administered to an experimental cell culture *in vitro* to prevent the growth of beta-lactam resistant bacteria. In certain other embodiments, a pharmaceutical composition described herein is administered to a mammal, including a human to prevent the growth of beta-lactam resistant bacteria *in vivo.*

In another aspect provided herein are methods of treating a bacterial infection, which method comprises administering to a subject a pharmaceutical composition comprising a pharmaceutical composition described herein. In some embodiments, the bacterial infection is an upper or lower respiratory tract infection, a urinary tract infection, an intra-abdominal infection, or a skin infection.

In some embodiments, the bacterial infection is acute bacterial skin and skin structure infections (ABSSSI), uncomplicated UTI (uUTI), complicated UTI (cUTI), complicated intraabdominal infections (cIAI), febrile neutropenia, community acquired bacterial pneumonia (CABP), hospital-acquired bacterial pneumonia (HABP), ventilator-associated bacterial pneumonia (VABP), or bacteremia. In some embodiments, the bacterial infection is acute bacterial skin and skin structure infections (ABSSSI). In some embodiments, the bacterial infection is uncomplicated UTI (uUTI). In some embodiments, the bacterial infection is complicated UTI (cUTI). In some embodiments, the bacterial infection is complicated intraabdominal infections (cIAI). In some embodiments, the bacterial infection is febrile neutropenia. In some embodiments, the bacterial infection is community acquired bacterial pneumonia (CABP). In some embodiments, the bacterial infection is hospital-acquired bacterial pneumonia (HABP). In some embodiments, the bacterial infection is ventilator-associated bacterial pneumonia (VABP). In some embodiments, the bacterial infection is bacteremia.

In some embodiments, the infection that is treated or prevented comprises a bacteria that includes *Elizabethkingia meningoseptica, Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides* 3452A homology group, *Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus* subsp. *hyicus, Staphylococcus haemolyticus, Staphylococcus hominis,* or *Staphylococcus saccharolyticus.*

In some embodiments, the infection that is treated or prevented comprises a bacteria that includes *Elizabethkingia meningoseptica* , *Pseudomonas aeruginosa, Pseudomonas fluorescens, Stenotrophomonas maltophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Acinetobacter calcoaceticus, Acinetobacter haemolyticus, Yersinia enterocolitica, Yersinia pestis, Yersinia pseudotuberculosis, Yersinia intermedia, Haemophilus influenzae, Haemophilus parainfluenzae, Haemophilus haemolyticus, Haemophilus parahaemolyticus, Helicobacter pylori, Campylobacter fetus, Campylobacter jejuni, Campylobacter coli, Vibrio cholerae, Vibrio parahaemolyticus, Legionella pneumophila, Listeria monocytogenes, Neisseria gonorrhoeae, Neisseria meningitidis, Moraxella, Bacteroides fragilis, Bacteroides vulgatus, Bacteroides ovalus, Bacteroides thetaiotaomicron, Bacteroides uniformis, Bacteroides eggerthii,* or *Bacteroides splanchnicus.*

### EXAMPLES

### Reference Example 1: In vitro efficacy of combination formulations of Compound 1 plus sulbactam.

To determine the ability of test compounds to potentiate the antibacterial activity of sulbactam (Cat# 1623670, USP, Rockville, MD) and sulbactam combinations against *Acinetobacter,* classic cell based broth microdilution MIC assays were employed. A panel of 51 Acinetobacter isolates with elevated sulbactam MICs were used. Of these isolates 40 were characterized and known to produce an OXA-23-like (Ambler Class D carbapenemase) and at least one additional beta-lactamase enzymes while the remaining 11 were uncharacterized. The assay was conducted in Cation Adjusted Mueller Hinton Broth (CAMHB, BD # 212322, BD Diagnostic Systems, Sparks, MD). Bacteria strains were grown for 3-5 hours in CAMHB broth. The following antibacterial test articles were added either alone or at fixed ratios to a microtiter plate in 2-fold serial dilutions in CAMHB, sulbactam, ampicillin (Cat# A9518-5G, Sigma, St. Louis, MO), cefepime (Cat# 1097636, USP, Rockville, MD). β-lactamase Inhibitors (BLI, e.g. Compound 1) were added at a fixed concentration of 4 µg/mL when relevant. Titration of antibacterial alone or in combination and +/- BLI with MIC readout indicates the potentiation the combinations have compared to the standalone articles (Table 1 and 2). Once the test articles were added, the plates were inoculated according to CLSI broth microdilution method. After inoculation the plates were incubated for 16-20 hours at 37 °C then the Minimal Inhibitory Concentration (MIC) of the test compound was determined visually.

Minimum inhibitory concentration (MIC) data for Sulbactam (SUL), Ampicillin (AMP), and combinations with Compound 1 against a panel of multi-drug resistant *Acinetobacter baumannii* are shown in Table 1. A 2:1 ratio of AMP: SUL (commercially available as the drug product Unasyn^{®}) was employed.

**Table 1. Minimum inhibitory concentrations (MIC, µg/mL) of Sulbactam (SUL), Ampicillin (AMP), and combinations with Compound 1 (fixed at 4 µg/mL). ND, not determined.**

| **Strain ID** | **Enzyme Content** | **SUL** | **AMP + SUL (2:1)** | **SUL + Compd 1 (at 4 µg/mL)** | **AMP + SUL (2:1) + Compd 1 (at 4 µg/mL)** |
|---|---|---|---|---|---|
| 0273 | ADC-25, OXA-23, OXA-66 | 32 | 16 | 8 | 16 |
| 0274 | TEM-1D, ADC-25, OXA-66, OXA-72 | 64 | 64 | 16 | 16 |
| 0275 | TEM-1D, ADC-25, OXA-23, OXA-66 | 64 | 64 | 16 | 16 |
| 0276 | ND | 8 | 8 | 8 | 16 |
| 0277 | TEM-1B, OXA-65, OXA-24 | 32 | 32 | 8 | 8 |
| 0278 | TEM-1D, ADC-25, OXA-23, OXA-66 | 32 | 64 | 8 | 8 |
| 0279 | TEM-1D, ADC-25, OXA-23, OXA-66 | 32 | 64 | 8 | 16 |
| 0280 | TEM-1D, ADC-25, OXA-66 | 128 | 128.1 | 4 | 16 |
| 0281 | TEM-1D, ADC-25, OXA-82 | 32 | 32 | 2 | 8 |
| 0282 | TEM-1D, ADC-25, OXA-23, OXA-66 | 32 | 64 | 16 | 32 |
| 0283 | TEM-1D, ADC-25, OXA-23, OXA-66 | 16 | 64 | 8 | 8 |
| 0284 | TEM-1B, OXA-65, OXA-24 | 32 | 64 | 4 | 8 |
| 0285 | TEM-1B, OXA-65, OXA-24 | 16 | 32 | 16 | 16 |
| 0286 | ADC-25, OXA-66, OXA-24 | 16 | 16 | 4 | 16 |
| 0287 | ADC-25, OXA-66, OXA-72 | 64 | 64 | 16 | 32 |
| 0288 | ADC-25, OXA-23, OXA-66 | 32 | 16 | 8 | 8 |
| 0289 | ADC-25, OXA-66, OXA-72 | 16 | 32 | 8 | 16 |
| 0290 | TEM-1D, ADC-25, OXA-23, OXA-66 | 32 | 64 | 8 | 16 |
| 0291 | TEM-1D, ADC-25, OXA-23, OXA-66 | 16 | 64 | 8 | 8 |
| 0292 | OXA-66, OXA-72 | 32 | 32 | 16 | 32 |
| 0293 | OXA-66, OXA-72 | 16 | 32 | 32 | 32 |
| 0294 | TEM-1B, OXA-23, OXA-65 | 16 | 32 | 16 | 16 |
| 0295 | TEM-1D, ADC-25, OXA-23, OXA-66 | 32 | 64 | 32 | 32 |
| 0296 | ADC-25, OXA-23, OXA-223 | 64 | 32 | 32 | 32 |
| 0297 | ADC-25, OXA-23, OXA-66 | 8 | 16 | 8 | 8 |
| 0298 | TEM-1D, ADC-25, OXA-66, OXA-237 | 32 | 64 | 8 | 8 |
| 0299 | PER-7, OXA-23, OXA-203 | 32 | 32 | 8 | 16 |
| 0300 | ADC-25, OXA-66 | 2 | 4 | 2 | 2 |
| 0301 | ADC-25, OXA-66, OXA-72 | 16 | 16 | 8 | 8 |
| 0302 | OXA-23, OXA-82 | 32 | 16 | 8 | 16 |
| 0303 | ADC-25, OXA-23, OXA-66 | 8 | 16 | 4 | 8 |
| 0304 | ADC-25, OXA-66, OXA-72 | 8 | 16 | 8 | 8 |
| 0305 | TEM-1B, OXA-65, OXA-24 | 8 | 16 | 2 | 4 |
| 0306 | TEM-1B, OXA-65, OXA-24 | 64 | 64 | 8 | 16 |
| 0307 | ADC-25, OXA-66, OXA-237 | 4 | 8 | 4 | 4 |
| 0308 | TEM-1D, ADC-25, OXA-71 | 32 | 32 | 4 | 4 |
| 0309 | OXA-23, OXA-82 | 8 | 16 | 8 | 16 |
| 0310 | OXA-23, OXA-82 | 32 | 32 | 16 | 16 |
| 0311 | ADC-25, OXA-23, OXA-82 | 16 | 32 | 16 | 16 |
| 0312 | OXA-69 | 4 | 4 | 2 | 2 |
| 0313 | TEM-1D, OXA-23, OXA-69 | 32 | 128 | 8 | 8 |
| 157834 | ND | 2 | 2 | 1 | 2 |
| 157842 | ND | 2 | 2 | 1 | 4 |
| 157876 | ND | 4 | 8 | 2 | 2 |
| 157901 | ND | 16 | 16 | 8 | 4 |
| 161750 | ND | 8 | 8 | 4 | 4 |
| 161752 | ND | 4 | 4 | 2 | 2 |
| 161755 | ND | 4 | 4 | 2 | 2 |
| 161756 | ND | 2 | 8 | 2 | 2 |
| 161760 | ND | 16 | 32 | 8 | 8 |
| 161762 | ND | 4 | 4 | 2 | 2 |
| **MIC₅₀** | | **16** | **32** | **8** | **8** |
| **MIC₉₀** | | **64** | **64** | **16** | **32** |

### Reference Example 2: In vitro efficacy of combination formulations of Compound 1 plus sulbactam in the presence of cefepime.

Minimum inhibitory concentration (MIC) data for Compound 1/cefepime (FEP)/Sulbactam (SUL)/ampicillin (AMP) combinations vs. comparators against a panel of multi-drug resistant *Acinetobacter baumannii* are shown in Table 2. MIC values for the indicated fixed ratios of the beta-lactam antibiotics are provided.

**Table 2. Minimum inhibitory concentrations (MIC, µg/mL) of Cefepime (FEP), Ampicillin (AMP), Sulbactam (SUL) and combinations with Compound 1 (fixed at 4 µg/mL). ND, not determined.**

| **Strain ID** | **Enzyme Content** | **FEP** | **FEP + Compd 1 (at 4 µg/mL)** | **FEP + SUL (1:1) + Compd 1 (at 4 µg/mL)** | **FEP + AMP + SUL (1:1:1) + Compd 1 (at 4 µg/mL)** |
|---|---|---|---|---|---|
| 0273 | ADC-25, OXA-23, OXA-66 | 32 | 32 | 8 | 8 |
| 0274 | TEM-1D, ADC-25, OXA-66, OXA-72 | 32 | 32 | 8 | 8 |
| 0275 | TEM-1D, ADC-25, OXA-23, OXA-66 | 32 | 64 | 8 | 8 |
| 0276 | ND | 128.1 | 128.1 | 16 | 16 |
| 0277 | TEM-1B, OXA-65, OXA-24 | 128.1 | 128 | 4 | 4 |
| 0278 | TEM-1D, ADC-25, OXA-23, OXA-66 | 32 | 32 | 8 | 16 |
| 0279 | TEM-1D, ADC-25, OXA-23, OXA-66 | 32 | 32 | 8 | 8 |
| 0280 | TEM-1D, ADC-25, OXA-66 | 16 | 16 | 4 | 4 |
| 0281 | TEM-1D, ADC-25, OXA-82 | 16 | 8 | 4 | 4 |
| 0282 | TEM-1D, ADC-25, OXA-23, OXA-66 | 64 | 64 | 16 | 16 |
| 0283 | TEM-1D, ADC-25, OXA-23, OXA-66 | 16 | 32 | 4 | 4 |
| 0284 | TEM-1B, OXA-65, OXA-24 | 32 | 32 | 4 | 4 |
| 0285 | TEM-1B, OXA-65, OXA-24 | 32 | 32 | 8 | 8 |
| 0286 | ADC-25, OXA-66, OXA-24 | 32 | 32 | 4 | 4 |
| 0287 | ADC-25, OXA-66, OXA-72 | 32 | 16 | 8 | 8 |
| 0288 | ADC-25, OXA-23, OXA-66 | 128 | 32 | 8 | 8 |
| 0289 | ADC-25, OXA-66, OXA-72 | 32 | 16 | 8 | 8 |
| 0290 | TEM-1D, ADC-25, OXA-23, OXA-66 | 32 | 32 | 16 | 8 |
| 0291 | TEM-1D, ADC-25, OXA-23, OXA-66 | 8 | 16 | 4 | 4 |
| 0292 | OXA-66, OXA-72 | 32 | 8 | 4 | 4 |
| 0293 | OXA-66, OXA-72 | 16 | 32 | 4 | 4 |
| 0294 | TEM-1B, OXA-23, OXA-65 | 32 | 32 | 8 | 8 |
| 0295 | TEM-1D, ADC-25, OXA-23, OXA-66 | 32 | 64 | 16 | 8 |
| 0296 | ADC-25, OXA-23, OXA-223 | 64 | 64 | 16 | 16 |
| 0297 | ADC-25, OXA-23, OXA-66 | 32 | 32 | 4 | 4 |
| 0298 | TEM-1D, ADC-25, OXA-66, OXA-237 | 32 | 32 | 4 | 8 |
| 0299 | PER-7, OXA-23, OXA-203 | 128 | 16 | 8 | 8 |
| 0300 | ADC-25, OXA-66 | 16 | 8 | 2 | 4 |
| 0301 | ADC-25, OXA-66, OXA-72 | 128.1 | 128.1 | 8 | 8 |
| 0302 | OXA-23, OXA-82 | 16 | 32 | 16 | 8 |
| 0303 | ADC-25, OXA-23, OXA-66 | 16 | 8 | 4 | 4 |
| 0304 | ADC-25, OXA-66, OXA-72 | 32 | 16 | 4 | 4 |
| 0305 | TEM-1B, OXA-65, OXA-24 | 16 | 16 | 2 | 4 |
| 0306 | TEM-1B, OXA-65, OXA-24 | 128 | 32 | 8 | 8 |
| 0307 | ADC-25, OXA-66, OXA-237 | 32 | 8 | 2 | 4 |
| 0308 | TEM-1D, ADC-25, OXA-71 | 16 | 8 | 2 | 4 |
| 0309 | OXA-23, OXA-82 | 64 | 16 | 8 | 8 |
| 0310 | OXA-23, OXA-82 | 32 | 32 | 8 | 8 |
| 0311 | ADC-25, OXA-23, OXA-82 | 128.1 | 128.1 | 8 | 16 |
| 0312 | OXA-69 | 32 | 32 | 2 | 4 |
| 0313 | TEM-1D, OXA-23, OXA-69 | 64 | 16 | 4 | 4 |
| 157834 | ND | 16 | 16 | 1 | 4 |
| 157842 | ND | 4 | 2 | 0.5 | 4 |
| 157876 | ND | 8 | 4 | 1 | 4 |
| 157901 | ND | 16 | 16 | 2 | 4 |
| 161750 | ND | 128 | 16 | 4 | 4 |
| 161752 | ND | 16 | 8 | 1 | 4 |
| 161755 | ND | 8 | 16 | 2 | 4 |
| 161756 | ND | 8 | 8 | 1 | 4 |
| 161760 | ND | 32 | 8 | 4 | 4 |
| 161762 | ND | 8 | 8 | 1 | 4 |
| **MIC₅₀** | | **32** | **32** | **4** | **4** |
| **MIC₉₀** | | **128** | **64** | **16** | **8** |

### Example 3: In vitro efficacy of combination formulations of Compound 1 plus Cefiderocol.

To determine the ability of Compound 1 to potentiate the antibacterial activity of cefiderocol (FDC) against β-lactamase producing strains of Enterobacteriaceae, classic cell based broth microdilution MIC assays were employed. A panel of 30 strains of characterized Enterobacteriaceae isolates produce Ambler class A, B, C, and D β-lactamase were used. The assay was conducted in Cation Adjusted Mueller Hinton Broth (CAMHB, BD # 212322, BD Diagnostic Systems, Sparks, MD) and in parallel in Iron Depleted Medium (IDM). IDM was prepared by adding 100 g of chelex (a chelating agent) to 1 L of sterile CAMBH and stirring for 2 hours. The chelex treated media was then filtered using a 0.2 µm filter and divalent Ca, Mg, and Zn are added back at relevant concentrations. The pH was adjusted to 7.2 using HCl and the media was filtered again to ensure sterility. This created a medium that was devoid of iron but retains appropriate concentration of other cations and was necessary for evaluating the activity of cefiderocol originating from its uptake by bacterial iron transporters. Bacteria strains were grown for 3-5 hours in CAMHB and IDM. Cefiderocol was added either to a microtiter plate in 2-fold serial dilutions in CAMHB and in parallel in IDM. Cefiderocol was tested alone and with β-lactamase Inhibitor (Compound 1) added at a fixed concentration of 4 µg/mL or 8 µg/mL. Titration of cefiderocol alone or ± BLI with MIC readout indicates the potentiation the combinations have compared to the antibacterial alone, while the potentiation seen in IDM vs CAMBH indicates the potentiation of cefiderocol under more physiologically-relevant conditions (shown in **Table 3).** Once the test articles were added the plates were inoculated according to CLSI broth microdilution method. After inoculation, the plates were incubated for 16-20 hours at 37°C then the Minimal Inhibitory Concentration (MIC) of the test compound was determined visually. This process was repeated in IDM only (not CAMHB) for cefiderocol alone and with compound 1 fixed at 4 µg/mL or 8 µg/mL in a panel of 40 MDR *Acinetobacter* (shown in **Table 4)** and for cefiderocol alone and with Compound **1** fixed at 4 µg/mL in a panel of 16 MDR *Pseudomonas aeruginosa* (shown in **Table 5).**

**Table 3. Minimum inhibitory concentrations (MIC, µg/mL) of Cefiderocol (FDC) alone and in combination with Compound 1 (fixed at 4 or 8 µg/mL) in β-tactamase expressing Enterobacteriaceae.**

| **Strain ID** | **Enzyme Content** | **FDC** | | **FDC + Compd 1 (fixed at 4 µg/mL)** | | **FDC + Compd 1 (fixed at 8 µg/mL))** | |
|---|---|---|---|---|---|---|---|
| | | **IDM** | **MHBII** | **IDM** | **MHBII** | **IDM** | **MHBII** |
| *E.coli* 3073 | AmpC, CTX-M3 | 0.5 | 8 | 0.125 | 2 | 0.125 | 2 |
| *E.coli* 128 | AmpC, SHV-12, TOHO-2, TEM-1 | 0.5 | 2 | 0.03 | 0.5 | 0.03 | 0.5 |
| *E. coli* SI-P026TC | CMY-2, TEM-1 | 0.125 | 0.25 | 0.03 | 0.06 | 0.03 | 0.06 |
| *E.coli* 3327 | TEM-1, SHV-12 | 0.125 | 0.5 | 0.03 | 0.125 | 0.03 | 0.125 |
| *K. pneumoniae* 847747 | CTX-M-2, KPC | 0.125 | 8 | 0.03 | 1 | 0.03 | 1 |
| *E. cloacae* CDC-93 | KPC | 0.5 | 1 | 0.03 | 1 | 0.03 | 0.5 |
| *E. coli* 786978 | KPC 2 | 0.25 | 4 | 0.125 | 0.25 | 0.06 | 0.125 |
| *K. pneumoniae* KPC261 | KPC-2 | 0.5 | 16 | 0.125 | 4 | 0.125 | 2 |
| *K. pneumoniae* 847204 | KPC-2 | 0.5 | 16 | 0.25 | 2 | 0.125 | 2 |
| *E.coli* CDC-114 | KPC-3 | 0.5 | 4 | 0.125 | 0.5 | 0.03 | 0.25 |
| *K. pneumoniae* KPC157 | KPC-3 | 0.5 | 8 | 0.125 | 4 | 0.125 | 2 |
| *E.coli* CDC-61 | KPC-3, TEM-1 | 0.125 | 0.25 | 0.06 | 0.25 | 0.03 | 0.25 |
| *E. cloacae* Entb239 | p99 AmpC, KPC-3, CTXM-15, GES-1, IMI-1 | 2 | 1 | 0.5 | 1 | 0.5 | 4 |
| *K. pneumoniae* 847375 | SHV-12, KPC-2 | 2 | 32 | 0.03 | 2 | 0.03 | 2 |
| *E. coli* 787112 | OXA 48 | 64 | 64 | 0.125 | 1 | 0.03 | 4 |
| *K. pneumoniae* KIRK | OXA-162 | 0.25 | 32 | 0.03 | 2 | 0.03 | 2 |
| *K. pneummoniae* CDC-140 | OXA-181 | 0.25 | 0.5 | 0.03 | 0.06 | 0.03 | 0.06 |
| *K. pneummoniae* CDC-34 | IMP | 0.125 | 2 | 0.125 | 2 | 0.125 | 1 |
| *E. cloacae* CDC-161 | IMP | 1 | 16 | 1 | 8 | 1 | 8 |
| *P. mirabilis* CDC-159 | NDM | 4 | 32 | 0.03 | 0.5 | 0.03 | 0.5 |
| *K. pneummoniae* CDC-41 | NDM | 8 | 128 | 0.5 | 32 | 0.25 | 16 |
| *K. pneummoniae* CDC-153 | NDM, OXA-232 | 0.06 | 32 | 0.03 | 1 | 0.03 | 0.5 |
| *E. coli* SI-152 | NDM-1 | 0.125 | 0.125 | 0.03 | 0.06 | 0.03 | 0.06 |
| *E. coli* GUE 131 | NDM-1 | 1 | 2 | 0.06 | 0.06 | 0.06 | 0.125 |
| *K. pneumoniae* OMAN 13 | NDM-1 | 2 | 32 | 0.06 | 2 | 0.06 | 4 |
| *E.coli* CDC-150 | NDM-5 | 4 | 128.1 | 0.5 | 32 | 0.5 | 32 |
| *K. pneummoniae* CDC-40 | VIM | 1 | 2 | 0.25 | 0.25 | 0.25 | 0.25 |
| *E. coli* SI-151 | VIM-1 | 0.125 | 0.5 | 0.03 | 0.125 | 0.03 | 0.5 |
| *E. coli* SI-155S | VIM-1 | 1 | 2 | 0.03 | 0.5 | 0.03 | 0.125 |
| *K. pneumoniae* SI-117 | VIM-1 | 4 | 16 | 0.03 | 0.25 | 0.03 | 1 |
| **MIC₅₀** | | 0.5 | **4** | **0.06** | **1** | **0.03** | **0.5** |
| **MIC₉₀** | | 4 | **32** | **0.5** | **4** | **0.25** | **4** |

**Table 4. Minimum inhibitory concentrations (MIC, µg/mL) of Cefiderocol (FDC) alone and in combination with Compound 1 (fixed at 4 or 8 µg/mL) in MDR-Acinetobacter conducted in IDM.**

| **Strain ID** | **FDC** | **FDC + Compd 1 (fixed at 4 µg/mL)** | **FDC + Compd 1 (fixed at 8 µg/mL)** |
|---|---|---|---|
| *A. baumannii* 0045 | 0.5 | 0.25 | 0.25 |
| *A. baumannii* 157070 | 0.25 | 0.25 | 0.25 |
| *A. baumannii* 157071 | 0.03 | 0.03 | 0.03 |
| *A. baumannii* 157072 | 0.25 | 0.25 | 0.25 |
| *A. baumannii* 157073 | 0.125 | 0.06 | 0.06 |
| *A. baumannii* 157074 | 0.06 | 0.06 | 0.06 |
| *A. baumannii* 157076 | 0.125 | 0.125 | 0.125 |
| *A. baumannii* 157078 | 0.125 | 0.125 | 0.125 |
| *A. baumannii* 157079 | 0.5 | 0.5 | 0.5 |
| *A. baumannii* 157865 | 4 | 0.25 | 0.25 |
| *A. baumannii* 161754 | 0.25 | 0.125 | 0.125 |
| *A. baumannii* 161761 | 0.125 | 0.06 | 0.06 |
| *A. baumannii* 161763 | 0.5 | 0.5 | 0.5 |
| *A. baumannii* 163169 | 0.03 | 0.03 | 0.03 |
| *A. baumannii* 163264 | 0.125 | 0.06 | 0.06 |
| *A. baumannii* 164890 | 0.125 | 0.06 | 0.06 |
| *A. baumannii* 164891 | 0.125 | 0.06 | 0.06 |
| *A. baumannii* 164899 | 0.25 | 0.06 | 0.06 |
| *A. baumannii* 165039 | 0.125 | 0.125 | 0.125 |
| *A. baumannii* ATCC 17978 | 0.25 | 0.25 | 0.25 |
| *A. baumannii* CDC-0033 | 0.5 | 0.125 | 0.125 |
| *A. baumannii* CDC-0035 | 0.25 | 0.25 | 0.25 |
| *A. baumannii* CDC-0036 | 1 | 0.5 | 0.5 |
| *A. baumannii* CDC-0037 | 4 | 0.5 | 0.5 |
| *A. baumannii* CDC-0052 | 8 | 0.5 | 0.25 |
| *A. baumannii* CDC-0056 | 0.125 | 0.125 | 0.25 |
| *A. baumannii* CDC-0063 | 0.5 | 0.125 | 0.125 |
| *A. baumannii* CDC-0070 | 0.25 | 0.125 | 0.125 |
| *A. baumannii* CDC-0078 | 0.5 | 0.06 | 0.06 |
| *A. baumannii* CDC-0083 | 1 | 0.5 | 0.5 |
| *A. baumannii* CDC-0088 | 8.1 | 0.5 | 0.25 |
| *A. baumannii* CDC-0101 | 0.06 | 0.06 | 0.06 |
| *A. baumannii* CDC-0102 | 1 | 0.25 | 0.25 |
| *A. baumannii* GM235 | 1 | 0.5 | 0.5 |
| *A. baumannii* GM236 | 0.03 | 0.03 | 0.03 |
| *A. baumannii* GM237 | 0.25 | 0.25 | 0.25 |
| *A. baumannii* GM238 | 0.25 | 0.125 | 0.125 |
| *A. baumannii* GM239 | 2 | 0.25 | 0.25 |
| *A. baumannii* NCTC 13301 | 1 | 0.25 | 0.25 |
| *A. baumannii* SI-141 | 4 | 0.25 | 0.25 |
| **MIC₅₀** | **0.25** | **0.125** | **0.125** |
| **MIC₉₀** | **4** | **0.5** | **0.5** |

**Table 5. Minimum inhibitory concentrations (MIC, µg/mL) of Cefiderocol (FDC) alone and in combination with Compound 1 (fixed at 4 µg/mL) in MDR-Pseudomonas conducted in IDM.**

| **Strain ID** | **FDC** | **FDC + Compd 1 (fixed at 4µg/mL)** |
|---|---|---|
| *P. aeruginosa* CDC-0054 | 0.5 | 0.059 |
| *P. aeruginosa* CDC-0064 | 0.5 | 0.059 |
| *P. aeruginosa* CDC-0090 | 0.125 | 0.059 |
| *P. aeruginosa* CDC-0095 | 0.059 | 0.125 |
| *P. aeruginosa* CDC-0103 | 4 | 0.125 |
| *P. aeruginosa* CDC-0108 | 0.25 | 0.25 |
| *P. aeruginosa* CDC-0110 | 8 | 0.059 |
| *P. aeruginosa* CDC-0111 | 0.059 | 0.25 |
| *P. aeruginosa* 40 | 4 | 0.125 |
| *P. aeruginosa* 451 | 4 | 0.059 |
| *P. aeruginosa* 635 | 0.5 | 0.125 |
| *P. aeruginosa* 152021 | 0.25 | 0.25 |
| *P. aeruginosa* 1013996 | 0.25 | 0.059 |
| *P. aeruginosa* 1131170 | 2 | 0.25 |
| *P. aeruginosa* 1121828 | 1 | 0.25 |
| *P. aeruginosa* 1179543 | 2 | 0.059 |
| **MIC₅₀** | **0.5** | **0.125** |
| **MIC₉₀** | **4** | **0.25** |

### Reference Example 4: Proposed clinical study of Compound 1 in combination with Sulbactam for the treatment of A. baumannii infections.

For possible combinations of Compound 1, sulbactam, ampicillin, and cefepime, a Phase I drug-drug interaction study (DDI) will be conducted to ensure safety and appropriate pharmacokinetics of the various combinations. Such studies would be conducted in two parts 1) a randomized, crossover drug-drug interaction study (part 1) and 2) a repeat-dose safety and PK study (part 2). Part 1 would consist of a single dose of the given combination, and Part 2 would be conducted over 7-10 days of repeat doses of the given combination and dosing regimen. Following completion of these DDI studies, epithelial lung fluid and renal impairment studies would be conducted, using doses and regimens derived from the results from the DDI studies.

Proposed drug ratios, doses, and dosing regimens are outlined as follows:
For pairing Compound 1 only with sulbactam, the combinations will be dosed over a 3-hour infusion period. The repeat dose study will be conducted with dosing every 6 hours over a 3-hour infusion period. Doses of the following are planned: 1 g sulbactam plus 1 g Compound 1; 1 g sulbactam plus 0.75 g Compound 1; 1 g sulbactam plus 0.5 g Compound 1; and 1 g sulbactam plus 0.25 g Compound 1.

For studies combining cefepime, sulbactam, and Compound 1, the combinations would be administered using a 3-hour infusion period. The repeat dose study will be conducted with dosing every 8 hours, using a 3-hour infusion period. Doses of the following combinations are planned: 2 g cefepime plus 1 g sulbactam plus 1 g Compound 1; 2 g cefepime plus 1 g sulbactam plus 0.75 g Compound 1; 2 g cefepime plus 1g sulbactam plus 0.5 g Compound 1; 2 g cefepime plus 1 g sulbactam plus 0.25 g Compound 1.

For studies combining cefepime, sulbactam, ampicillin, and Compound 1, the combinations will be administered using infusion times of both 2 hours and 3 hours. The repeat dose study will be conducted with dosing every 6 hours. The following doses are planned: 1 g cefepime plus 0.5 g Compound 1 plus 1 g ampicillin, plus 0.5 g sulbactam; 1 g cefepime plus 0.5 g Compound 1 plus 2 g ampicillin plus 1 g sulbactam.

### Example 5: Proposed clinical study of Compound 1 in combination with Cefiderocol for the treatment of MDR-Enterobacteriaceae infections.

Combinations of cefiderocol and Compound 1 will require a Phase I drug-drug interaction study to safety and appropriate pharmacokinetics of the combination of drugs. Such studies would be conducted in two parts 1) a randomized, crossover drug-drug interaction study (part 1) and 2) a repeat-dose safety and PK study (part 2). Part 1 would consist of a single dose of the given combination, and Part 2 would be conducted over 7-10 days of repeat doses of the given combination and dosing regimen. Following completion of these DDI studies, epithelial lung fluid and renal impairment studies would be conducted, using doses and regimens derived from the results from the DDI studies.

Combination dosing of cefiderocol and Compound 1 would be administered using both 2-hour and 3-hour infusion periods. The repeat-dose study will be conducted with infusions given every 8 hours. The following doses are planned: 2 g cefiderocol plus 0.5 g Compound 1; 2 g cefiderocol: 0.25 g Compound 1; and 2 g cefiderocol plus 0.125 g Compound 1.

### Reference Example 6: Proposed clinical study of Compound 1 in combination with Cefepime for Patients with cUTI

This study is a Phase 3, randomized, multicenter, double-blind, double dummy, active-controlled noninferiority study to evaluate the efficacy, safety, and tolerability of cefepime/Compound 1, a pharmaceutically acceptable salt, a solvate, or a pharmaceutically acceptable salt and solvate thereof, compared with meropenem in adult patients with cUTI, including pyelonephritis (AP).

Patients will be randomized in a 2:1 ratio to receive study treatment with either cefepime/ Compound 1 (2 g/0.5 g IV q8h) or the active comparator, meropenem (1 g IV q8h).

Patients will be stratified by the type of infection (AP only versus complicated lower UTI with or without AP) and region (North America and Western Europe versus Eastern Europe versus rest of the world). At least 30% of the population will have AP.

Study treatment will be administered IV for 7 days and will be considered completed after the third dose on Study Day 7. All patients (who do not require dosing adjustments for renal dysfunction) will receive at least 19 doses and no more than 21 doses of active study drug.

Patients with bacteremia at study entry may have their treatment extended up to 10 days at the Investigator's discretion. Patients will be admitted at screening and remain at the study site or hospital for the duration of the IV treatment period and through the EOT assessment. The EOT visit will be performed no more than 24 hours after the last dose of IV study drug. Patients may be discharged from the study site after the EOT assessment if clinically stable and at the Investigator's discretion.

Patients must return to the study site for the TOC assessment (Study Days 12 to 17) and the LFU assessment (Study Days 21 to 28).

### Choice and Dosing of Comparator

Meropenem has been selected as the comparator because it has demonstrated efficacy against gram-negative pathogens isolated in cUTIs, including pyelonephritis. Although in the US it is approved for other indications, including complicated intra-abdominal infections, skin and soft tissue structure infections and bacterial meningitis in pediatric patients it is not approved for use in cUTI in the US. However, meropenem is approved for cUTI in other geographic regions and was used as a comparator in the plazomicin pivotal cUTI study supporting approval. Furthermore, carbapenems are the drugs of choice against ESBL-producing gram-negative pathogens, especially in serious infections. As such, it is an appropriate comparator to use when evaluating a new drug designed to treat infections due to bacteria harboring serine-β-lactamases.

Meropenem 1g q8h will be given as an active comparator. In the US and elsewhere, meropenem is approved at doses of 0.5 or 1 g q8h (given by intravenous infusion over 15 to 30 minutes), depending on the type of infection. The Summary of Product Characteristics indicates that doses of 0.5 or 1 g meropenem q8h is appropriate for cUTI. In order to ensure the best therapy against pathogens that are more difficult to treat, the dose of 1 g has been chosen. Although meropenem is not approved in the US for cUTI, the US prescribing information indicates that 1 g meropenem q8h is appropriate for the treatment of patients with cIAIs and skin and soft tissue structure infections due to *P aeruginosa,* which is consistent with the goal to use a higher dose (i.e., 1 g) in more difficult-to-treat infections.

### Dose Administration and Adjustments

Each patient will receive a 30-minute infusion and a 2-hour infusion, once every 8 hours (q8h). If only one line is available for IV medication administration, the 30-minute infusion should be administered prior to the 2 hour infusion. Patients randomized to cefepime/Compound 1 will receive meropenem placebo (0.9% saline) infused over 30 minutes IV immediately followed by cefepime/Compound 1 (2 g/0.5 g IV q8h) infused over 2 hours. Patients randomized to receive meropenem will receive meropenem 1g IV infused over 30 minutes, immediately followed by placebo (0.9% saline) infused over 2 hours. The 30 minute and 2 hour infusion may be administered concurrently if two, separate lines are used.

### Treatment Duration

All patients will receive at least 7 days of IV treatment. All patients will be considered to complete dosing after their last dose on day 7. All patients (who do not require dosing adjustments for renal dysfunction) will receive at least 19 doses and no more than 21 doses of active study drug. Patients with bacteremia at study entry may have their treatment extended up to 10 days at the Investigator's discretion.

There is no oral stepdown therapy, and patients are not permitted to continue oral antibacterials at home after completion of IV study treatment.

### Primary Objective and Efficacy Endpoints

The primary objective of this non-inferiority study is to assess the efficacy of cefepime/Compound 1 compared with meropenem with respect to both per-patient microbiologic eradication and symptomatic resolution of all UTI-core signs and symptoms (or return to pre-morbid baseline) at the test of cure (TOC) visit. The TOC visit will occur 5-10 days after end of therapy for patients who do not have bacteremia at baseline and at least 2 days and no more than 7 days for patients who have bacteremia and have therapy extended. The primary analysis will be performed in the microbiologic ITT (microITT) population.

The response rate will be calculated for each treatment group as the number of successes divided by the total number of patients (success + failure + indeterminate). To be a success, a patient must be a success for both microbiologic and clinical outcomes. Patients with a failure in either microbiologic or clinical outcomes will be considered a failure. Patients with success in one outcome, but indeterminate outcome in the other will be considered to have a primary outcome of indeterminate.

### Microbiologic Response

Before receipt of study treatment, a urine specimen will be obtained from all patients for culture and for in vitro antibacterial susceptibility testing. Patients with an indwelling catheter should have urine samples collected following the placement of a new catheter, or if the indwelling catheter cannot be removed, aseptic techniques should be used through a properly disinfected collection port. Patients with indwelling catheter should be randomized only if they are expected to permanently discontinue use of the catheter prior to Study Day 5.

Microbiological outcome per-patient will be assessed in a blinded manner programmatically. It is based on outcome per-pathogen isolated at the initial visit (considered as causative) and on the isolation of a pathogen at TOC based on the definitions in Table 6. For patients with more than 1 pathogen present at baseline all pathogens must be eradicated to be classified as a microbiologic success.

**Table 6 Microbiological Response Criteria**

| **Microbiological Response** | **Definition** |
|---|---|
| Eradication | Demonstration that the bacterial pathogen found at study entry (≥10⁵ CFU/mL is eradicated to <10³ CFU/mL |
| Persistence | Demonstration that the bacterial pathogen found at study entry (≥ 10⁵ CFU/mL) grows ≥10³ CFU/mL |
| Indeterminate microbiological response | Repeat cultures are not performed |

| | |
|---|---|
| CFU=colony-forming unit. | |

### Symptomatic Clinical Response

Screening assessments include a Daily Patient Symptom Questionnaire (DPSQ) performed at baseline and a Pre-morbid Patient Symptom Questionnaire (PPSQ). The DPSQ will be administered at baseline, daily while on study therapy and at EOT, TOC and LFU visits to determine the presence and intensity of cUTI signs and symptoms. The PPSQ will be administered once at Baseline to determine whether a patient normally experiences urinary tract symptoms (i.e., in the absence of a UTI) that may be attributable to other disease processes (e.g., Benign Prostatic Hypertrophy).

The DPSQ and the PPSQ are formal questionnaires that will be administered by trained study center staff. The questions will be read out loud verbatim. The administrator is not permitted to interpret the questions for the patient, further explain the questions to the patient or comment on the patient response. The DPSQ obtained at the TOC visit will be programmatically compared to both the DPSQ obtained at baseline as well as the PPSQ to determine whether all core cUTI symptoms (dysuria urgency, frequency, flank pain/tenderness, suprapubic pain/tenderness) have resolved and or returned to the patient's pre-morbid (i.e., prior to the onset of the current cUTI) state in order to determine the symptomatic clinical response as defined in Table 7.

**Table 7: Symptomatic Clinical Response Criteria**

| **Response Category** | **Definition** | |
|---|---|---|
| Symptomatic Clinical success | Both of the following have occurred: | |
| | | • Resolution (or return to pre-morbid state) of the core symptoms of cUTI (dysuria, frequency, urgency, suprapubic/pelvic pain, and flank pain) present at study entry |
| | | • No new core cUTI symptoms have developed and no symptoms have worsened |
| Symptomatic Clinical failure | Occurrence of 1 or more of the following: | |
| | | • Confirmed persistence of at least 1 symptom (ie non-resolution or not having returned to pre-morbid state) of the core symptoms of cUTI (dysuria, frequency, urgency, suprapubic/pelvic pain, and flank pain) |
| | | • Development of 1 or more core symptoms of cUTI not present at baseline |
| | | • Death |
| Indeterminate symptomatic clinical response | Cases where the symptomatic clinical response could not be assessed | |

The definitions for symptomatic clinical response are based on FDA guidance. Notably, the proposed criteria are independent of post randomization events and are not impacted by receipt of an additional antibiotic to treat cUTI (i.e., due to deterioration in clinical status or premature discontinuation of study drug) prior to TOC. Furthermore, symptomatic clinical failure at the End of Therapy Visit (EOT) (within 24 hours after the last dose of study drug) is not a criterion for failure at TOC.

### Other Efficacy Endpoints

Secondary efficacy endpoints include evaluation of cefepime/Compound 1 versus meropenem in regards to microbiologic (per-patient and per pathogen) outcomes and clinical outcomes in several analysis populations (i.e., microITT, CE, ME) and at various timepoints (i.e., EOT, TOC and Late Follow Up (LFU; Study day 21-28).

In addition, outcomes by baseline MIC and among cefepime resistant pathogens will be evaluated. Post baseline cultures will be assessed for increasing MIC to study drug (indicated by the organism displaying a ≥4-fold higher MIC to study drug received in a urine or blood culture obtained at EOT or later compared to baseline), super infections (isolation of a new pathogen from the urine during study treatment that requires additional therapy) and new infections (isolation of a new pathogen from the urine during after completion of study treatment that requires additional therapy).

### Statistical Plan

An independent Data Safety Monitoring Board (DSMB) will be formed to monitor interim safety data during the study. Procedures for DSMB reviews/meetings will be defined in the DSMB charter. The DSMB will review applicable data at scheduled timepoints during the study as defined in the charter.

Additional data may be requested by the DSMB. The DSMB will review grouped and unblinded (upon request) data in the closed session only. As an outcome of each review/meeting, the DSMB will make a recommendation as to the advisability of proceeding with study treatment administration, and to continue, modify, or terminate this study.

Ongoing data to be reviewed by the DSMB as well as VenatorRx and CRO medical monitors includes Adverse Event Monitoring (from consent through the LFU), Clinical Laboratory Assessments (at screening/baseline, Study day 4, Study day 8 (if still on study therapy), at EOT, at TOC and at LFU), and Vital Signs (at screening/baseline, daily while on study therapy, at EOT, at TOC and at LFU). Sites will be asked to collect ECGs as part of screening and on day 4 immediately after completion of the cefepime/Compound 1 or cefepime placebo infusion (and after study state has been achieved). Investigators should review all ECGs, follow up any abnormalities and report any significant findings as AEs if AE criteria are met. ECG results will not be collected or centrally analyzed a priori but in the event of any cardiac events, the site will have the ECGs available for review in the study record.

### Reference Example 7: Proposed clinical study of Compound 1 in combination with Cefepime for patients with infections caused by resistant pathogens

This study will be a prospective, Phase 3, multicenter, randomized, open-label, active-controlled study of cefepime/Compound 1 therapy (versus BAT (Best Available Therapy)) in patients with cUTI, cIAI, or bacteremia (not related to cIAI or cUTI; patients with cUTI or cIAI may have concurrent bacteremia) caused by resistant pathogens. For the purposes of this study, a resistant pathogen is an isolate of Enterobacteriaceae or Pseudomonas aeruginosa that demonstrates resistance to a carbapenem. The primary objective of this study is to estimate the response to cefepime/Compound 1 and BAT at Test of Cure (TOC) in the treatment of selected serious infections caused by carbapenem-resistant Enterobacteriaceae spp and Pseudomonas aeruginosa.

This study aims to recruit as many eligible patients as possible in the time period (24 months). This study is expected to recruit approximately 75 patients (approximately 50 in the cefepime/Compound 1 combination therapy group and approximately 25 in the group receiving BAT. Cefepime hydrochloride for injection (2 g/vial) and Compound 1, a pharmaceutically acceptable salt, a solvate, or a pharmaceutically acceptable salt and solvate thereof (0.5 g/vial) will each be reconstituted independently, combined in a single IV bag, and diluted to a total volume of 250 mL with 0.9% sodium chloride for injection. Cefepime/Compound 1 will be administered over a 2-hour period at a rate of 2.1 mL/min. For patients with cIAI who are receiving cefepime/ Compound 1 metronidazole 0.5 g will be administered immediately following each cefepime/ Compound 1 infusion. Metronidazole for injection will be administered as an IV infusion over 30 minutes. Patients randomized to receive the investigator-determined BAT will receive IV doses based on the investigator's standard of care and local label recommendation. Treatment will be continued for a period of time (5 full days to 14 full days) deemed appropriate by the investigator based upon the demonstration of clinical improvement.

### Reference Example 8: In Vivo Thigh Infection Model for Dose Determination

The 2 g:0.5 g dose of cefepime: Compound 1 which is planned for clinical efficacy studies is supported by neutropenic murine thigh infection model studies using 30 cefepime-resistant clinical isolates of Enterobacteriaceae and *P. aeruginosa.*

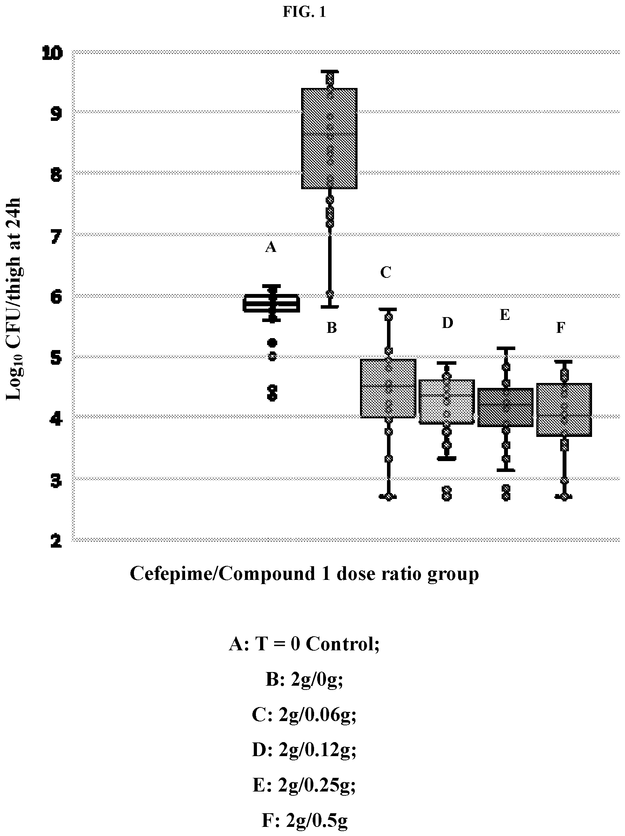

In these studies, the human simulated regimen (HSR) of 2 g cefepime alone q8h was ineffective at 24 h (FIG. 1, B), allowing approximately 2.7 logic CFU/thigh growth on average compared to the T=0 control (FIG. 1, A). Cefepime activity was maximally rescued when Compound 1 was administered at an HSR of 0.5 g on the same q8h schedule as cefepime: this regimen attained on average 1.7 logic bacterial killing relative to the initial bacterial burden (FIG. 1, F). This extent of killing *in vivo* exceeds the stasis to 1 log₁₀ kill target for efficacy that predicts clinical efficacy in patients with cUTI. Whereas for several of the tested strains, significant rescue of cefepime activity was observed at lower Compound 1 doses in combination with cefepime (FIG. 1, C, D, and E), the cefepime/Compound 12 g:0.5 g HSR reliably protected cefepime bactericidal activity across all 30 tested strains, regardless of their β-lactamase content.

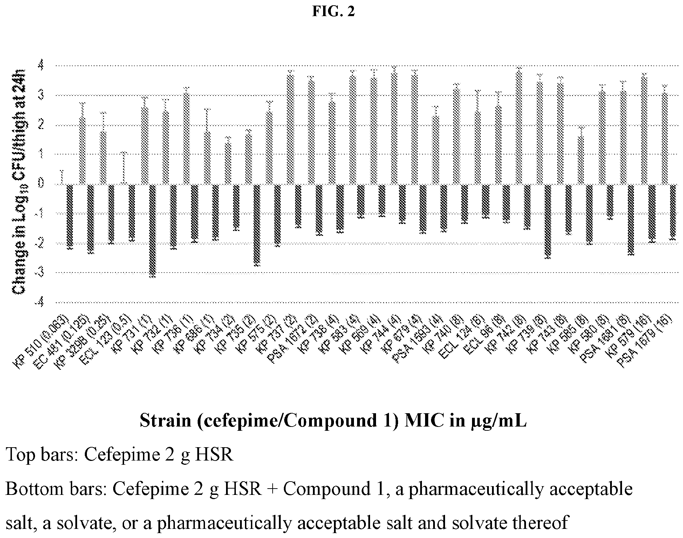

When dosed to mice using an HSR of cefepime 2 g:Compound 1 0.5 g, the combination exerted at least 1-log₁₀ killing against each of the 30 tested strains, regardless of their cefepime/Compound 1 MIC (FIG. 2). No evidence was seen of adaptive resistance, which would be evident as growth of the infecting strain following dosing with the combination. The neutropenic murine thigh infection model data therefore supports the cefepime 2 g:Compound 1 0.5 g dose for clinical studies because it reliably protected cefepime bactericidal activity across all tested strains, regardless of their β-lactamase content or cefepime/Compound 1 MIC, up to the highest tested MIC of 16 µg/mL.

### Reference Example 9: In vitro efficacy of triple combination

For a more comprehensive assessment of the ability of test compounds to potentiate sulbactam in triple combinations with β-lactams additional MIC assays were utilized. The antimicrobial activity of cefepime and meropenem were evaluated in the presence of the Compound 1 and/or sulbactam against a set of 141 *Acinetobacter baumannii-calcoaceticus* species complex clinical isolates exhibiting elevated MIC values for ampicillin-sulbactam.

**Table 8. Minimal Inhibitory Concentration (MIC, µg/mL) of Compound 1 (4 µg/mL) in combination with sulbactam (SUL), or sulbactam and either meropenem (MEM) or cefepime (FEP) vs. sulbactam alone, or ampicillin-sulbactam (AMP-SUL), in 141 globally collected clinical isolates of Acinetobacter baumannii.**

| **Strain** | **FEP** | **FEP + Cmpd 1** | **FEP + SUL (1:1) + Cmpd 1** | **MEM** | **MEM + Cmpd 1** | **MEM + SUL (1:1) + Cmpd 1** | **SUL** | **SUL + Cmpd 1** | **AMP-SUL (2:1)** |
|---|---|---|---|---|---|---|---|---|---|
| 1042182 | 32 | 32 | 8 | 64 | 64 | 16 | 16 | 16 | 32 |
| 1040917 | >512 | >256 | 32 | 128 | 128 | 64 | 128 | 64 | >64 |
| 1040916 | 128 | 128 | 16 | 128 | 64 | 16 | 32 | 16 | >64 |
| 1040901 | 256 | 64 | 16 | 64 | 64 | 8 | 64 | 16 | >64 |
| 1040900 | 64 | 64 | 8 | 64 | 64 | 8 | 8 | 16 | 64 |
| 1039518 | 64 | 32 | 8 | 64 | 64 | 8 | 16 | 8 | 32 |
| 1039515 | 64 | 64 | 16 | 128 | 128 | 16 | 32 | 16 | 64 |
| 1039514 | 256 | 64 | 16 | 128 | 128 | 16 | 16 | 32 | 32 |
| 1039502 | >512 | >256 | 32 | 64 | 128 | 16 | 64 | 64 | >64 |
| 1038439 | 128 | 128 | 8 | 0.25 | 0.25 | 0.25 | 16 | 16 | 32 |
| 1038418 | 128 | 128 | 16 | 0.25 | 0.25 | <=0.12 | 16 | 16 | 32 |
| 1037747 | >512 | 16 | 4 | 256 | 256 | 8 | 32 | 8 | >64 |
| 1037738 | 32 | 32 | 8 | >256 | >256 | 16 | 16 | 8 | 32 |
| 1037732 | 32 | 32 | 8 | 256 | 256 | 16 | 16 | 16 | 64 |
| 1037731 | 256 | 8 | 4 | 0.5 | 0.5 | 0.25 | 16 | 4 | 32 |
| 1037714 | 4 | 2 | 2 | 256 | 256 | 8 | 8 | 8 | 64 |
| 1037618 | 256 | 16 | 4 | 1 | 1 | 0.5 | 64 | 16 | >64 |
| 1037593 | 256 | 16 | 8 | 256 | 256 | 16 | 32 | 16 | 64 |
| 1037479 | >512 | 8 | 4 | 256 | 128 | 8 | 128 | 16 | >64 |
| 1037462 | 256 | 64 | 8 | 128 | 128 | 8 | 16 | 16 | 64 |
| 1037294 | 256 | 128 | 32 | 128 | 128 | 32 | 64 | 64 | >64 |
| 1037256 | 32 | 32 | 8 | 64 | 64 | 8 | 16 | 16 | 64 |
| 1037251 | 512 | 32 | 8 | 64 | 64 | 16 | 32 | 16 | >64 |
| 1037211 | 512 | 32 | 16 | 2 | 1 | 1 | 64 | 16 | 64 |
| 1037193 | 32 | 16 | 8 | >256 | 256 | 8 | 16 | 16 | 32 |
| 1036921 | 32 | 16 | 2 | 1 | 1 | 0.5 | 64 | 2 | 64 |
| 1036882 | 16 | 32 | 8 | 256 | 256 | 16 | 32 | 16 | 64 |
| 1036859 | 256 | 128 | 16 | 64 | 128 | 16 | 32 | 16 | 64 |
| 1036781 | 32 | 16 | 8 | >256 | 256 | 16 | 32 | 16 | >64 |
| 1036768 | >512 | 8 | 4 | 8 | 4 | 2 | 32 | 4 | 64 |
| 1036734 | 128 | 64 | 8 | 64 | 32 | 8 | 32 | 16 | 64 |
| 1036068 | 64 | 64 | 16 | 64 | 64 | 16 | 32 | 16 | >64 |
| 1036045 | 64 | 32 | 16 | 64 | 128 | 16 | 32 | 16 | 64 |
| 1036023 | 128 | 64 | 8 | 128 | 128 | 16 | 64 | 16 | 64 |
| 1035900 | 128 | 64 | 16 | 128 | 128 | 16 | 32 | 64 | >64 |
| 1035825 | 64 | 32 | 8 | 64 | 64 | 8 | 32 | 8 | 64 |
| 1035808 | 256 | 16 | 8 | 16 | 16 | 8 | 32 | 16 | 64 |
| 1035794 | 16 | 16 | 8 | 32 | 32 | 32 | 64 | 64 | >64 |
| 1035788 | 32 | 32 | 8 | 64 | 64 | 8 | 32 | 16 | 64 |
| 1035144 | 256 | 256 | 32 | 256 | 256 | 32 | 32 | 64 | >64 |
| 1034962 | 512 | 64 | 16 | 64 | 64 | 16 | 32 | 16 | 64 |
| 1034951 | 256 | 128 | 16 | 128 | 128 | 32 | 32 | 32 | >64 |
| 1034931 | 128 | 16 | 2 | 128 | 64 | 4 | 16 | 4 | 32 |
| 1034925 | 64 | 32 | 8 | 64 | 32 | 8 | 32 | 8 | 64 |
| 1034916 | 256 | 32 | 8 | 64 | 64 | 16 | 64 | 16 | >64 |
| 1034913 | 128 | 64 | 8 | 64 | 64 | 16 | 32 | 16 | >64 |
| 1034912 | 64 | 32 | 16 | 64 | 64 | 16 | 32 | 32 | 64 |
| 1034880 | 64 | 64 | 16 | 128 | 64 | 32 | 32 | 16 | 64 |
| 1034048 | 128 | 128 | 32 | 256 | 256 | 16 | 32 | 32 | 64 |
| 1033979 | 256 | 64 | 16 | 256 | 256 | 32 | 64 | 64 | >64 |
| 1033340 | 128 | 64 | 8 | 256 | 128 | 8 | 16 | 8 | 32 |
| 1032761 | 256 | 128 | 32 | 256 | 256 | 64 | 64 | 64 | >64 |
| 1032669 | 64 | 64 | 8 | 64 | 64 | 16 | 16 | 16 | 32 |
| 1031566 | 128 | 64 | 16 | 128 | 64 | 16 | 32 | 16 | 64 |
| 1031238 | 512 | 256 | 32 | 256 | 256 | 64 | 32 | 32 | >64 |
| 1031211 | 256 | 256 | 32 | 64 | 128 | 16 | 32 | 32 | >64 |
| 1031191 | 64 | 64 | 16 | 64 | 64 | 16 | 16 | 16 | 32 |
| 1031177 | 128 | 64 | 16 | 128 | 128 | 16 | 16 | 32 | 64 |
| 1031167 | 256 | 256 | 32 | 256 | 256 | 32 | 64 | 32 | >64 |
| 1031136 | 256 | 64 | 32 | 128 | 128 | 32 | 32 | 64 | >64 |
| 1030945 | 128 | 128 | 32 | 128 | 128 | 32 | 32 | 32 | >64 |
| 1030928 | >512 | >256 | 32 | 128 | 128 | 32 | 64 | 64 | >64 |
| 1030427 | 32 | 32 | 8 | 256 | 128 | 16 | 16 | 16 | 32 |
| 1029913 | 32 | 32 | 8 | 32 | 32 | 8 | 16 | 16 | 64 |
| 1029770 | 256 | 256 | 16 | >256 | 256 | 32 | 32 | 32 | >64 |
| 1028193 | 32 | 64 | 16 | >256 | >256 | 32 | 64 | 32 | 64 |
| 1027748 | 32 | 64 | 8 | 32 | 32 | 8 | 8 | 8 | 32 |
| 1027603 | 64 | 32 | 16 | 64 | 32 | 16 | 8 | 4 | 32 |
| 1027590 | 256 | 256 | 32 | 256 | 128 | 32 | 32 | 32 | >64 |
| 1027234 | 64 | 64 | 8 | 64 | 64 | 8 | 8 | 8 | 32 |
| 1026992 | 256 | 32 | 16 | 32 | 64 | 8 | 16 | 16 | 32 |
| 1026990 | 256 | 64 | 8 | 256 | 256 | 8 | 16 | 16 | 64 |
| 1024215 | 32 | 16 | 8 | 128 | 128 | 16 | 16 | 16 | 32 |
| 1024119 | 32 | 16 | 4 | 4 | 2 | 1 | 32 | 4 | 64 |
| 1023329 | 128 | 128 | 8 | 64 | 64 | 8 | 16 | 16 | 32 |
| 1023207 | 32 | 16 | 8 | 32 | 32 | 8 | 16 | 8 | 32 |
| 1023074 | 128 | 128 | 16 | 32 | 32 | 8 | 32 | 32 | 32 |
| 1023034 | 256 | 256 | 8 | 64 | 64 | 8 | 16 | 8 | 64 |
| 1023031 | 256 | 128 | 64 | >256 | >256 | 64 | 128 | 64 | 64 |
| 1019086 | 512 | 256 | 64 | 256 | 128 | 32 | 128 | 64 | >64 |
| 1018851 | 256 | 256 | 32 | 256 | 256 | 32 | 64 | 32 | >64 |
| 1018807 | 128 | 128 | 16 | 64 | 64 | 16 | 32 | 16 | 64 |
| 1018806 | 512 | 32 | 4 | 128 | 8 | 2 | 64 | 8 | >64 |
| 1018804 | 256 | 256 | 16 | 128 | 128 | 16 | 32 | 32 | 64 |
| 1018802 | 256 | 128 | 32 | 256 | 128 | 32 | 64 | 64 | >64 |
| 1018801 | >512 | >256 | 32 | 256 | 128 | 16 | 64 | 64 | >64 |
| 1018109 | 2 | 2 | 1 | 0.25 | 0.25 | 0.25 | 4 | 2 | 32 |
| 1017679 | 128 | 64 | 16 | 128 | 128 | 16 | 32 | 16 | >64 |
| 1016836 | 64 | 64 | 16 | 128 | 128 | 16 | 32 | 16 | 32 |
| 1016545 | 128 | 256 | 16 | 128 | 128 | 16 | 16 | 16 | 64 |
| 1014890 | 256 | 128 | 16 | 128 | 128 | 32 | 32 | 16 | >64 |
| 1014878 | 128 | 128 | 8 | 64 | 64 | 8 | 32 | 16 | >64 |
| 1014870 | 256 | 256 | 32 | 256 | 256 | 64 | 128 | 64 | >64 |
| 1014866 | 128 | 128 | 8 | 64 | 64 | 8 | 32 | 16 | 64 |
| 1014815 | 256 | 128 | 32 | 256 | 256 | 64 | 64 | 32 | 64 |
| 1014646 | 64 | 32 | 4 | 64 | 32 | 4 | 16 | 4 | 64 |
| 1014143 | 32 | 16 | 4 | 8 | 8 | 4 | 32 | 4 | 64 |
| 1013322 | 128 | 64 | 8 | 64 | 64 | 8 | 16 | 16 | 32 |
| 1013055 | 256 | 8 | 1 | 32 | 2 | 0.5 | 16 | 2 | 32 |
| 1013035 | 32 | 16 | 2 | 256 | 128 | 4 | 16 | 4 | 64 |
| 1013022 | 64 | 16 | 4 | 256 | 256 | 4 | 8 | 8 | 32 |
| 1011959 | 64 | 32 | 8 | 64 | 64 | 16 | 16 | 16 | 32 |
| 1010950 | >512 | 32 | 8 | 128 | 16 | 4 | 128 | 8 | >64 |
| 1010383 | 128 | 128 | 16 | 128 | 128 | 16 | 32 | 32 | 64 |
| 1010331 | >512 | 256 | 64 | 64 | 32 | 16 | 128 | 256 | >64 |
| 1010245 | 64 | 64 | 16 | 64 | 64 | 16 | 16 | 16 | 32 |
| 1008756 | 512 | 256 | 32 | 128 | 128 | 32 | 64 | 64 | >64 |
| 1008730 | 64 | 128 | 32 | 128 | 128 | 32 | 32 | 32 | 64 |
| 1008510 | 64 | 32 | 8 | 256 | 256 | 8 | 32 | 8 | >64 |
| 1008508 | 128 | 64 | 8 | >256 | 256 | 16 | 16 | 8 | >64 |
| 1007660 | 128 | 128 | 16 | 128 | 128 | 32 | 32 | 32 | >64 |
| 1005290 | 256 | 64 | 16 | 128 | 128 | 16 | 16 | 16 | 32 |
| 1005265 | 128 | 128 | 16 | 128 | 128 | 32 | 32 | 32 | 64 |
| 1004867 | 256 | 128 | 32 | 128 | 128 | 32 | 64 | 64 | >64 |
| 1003787 | >512 | 8 | 4 | >256 | 256 | 16 | 32 | 16 | >64 |
| 1003662 | 512 | 256 | 16 | 128 | 128 | 16 | 16 | 16 | 64 |
| 1003557 | 64 | 16 | 4 | 256 | 256 | 4 | 8 | 4 | 32 |
| 1002960 | 128 | 128 | 32 | >256 | >256 | 64 | 128 | 128 | >64 |
| 1002952 | 64 | 32 | 16 | >256 | >256 | 32 | 64 | 64 | >64 |
| 1002688 | 64 | 64 | 16 | 128 | 64 | 16 | 16 | 16 | 64 |
| 1002247 | >512 | 256 | 64 | 128 | 128 | 8 | 64 | 32 | >64 |
| 1002208 | 64 | 32 | 8 | 64 | 64 | 16 | 16 | 16 | 32 |
| 1001611 | 16 | 16 | 4 | 32 | 32 | 4 | 16 | 8 | 32 |
| 1001143 | 128 | 128 | 32 | 128 | 128 | 32 | 32 | 32 | 32 |
| 1001007 | 256 | 128 | 16 | 256 | 256 | 16 | 32 | 32 | >64 |
| 999343 | 256 | 128 | 32 | 128 | 128 | 16 | 32 | 32 | 64 |
| 999306 | 8 | 16 | 2 | 2 | 1 | 0.5 | 8 | 2 | 32 |
| 998907 | 8 | 8 | 4 | 16 | 16 | 4 | 32 | 4 | 64 |
| 998898 | 512 | >256 | 64 | 32 | 32 | 8 | 64 | 128 | >64 |
| 998532 | 64 | 32 | 4 | 256 | 256 | 16 | 16 | 8 | 64 |
| 998107 | 128 | 64 | 8 | 64 | 64 | 8 | 16 | 16 | 32 |
| 997574 | 16 | 8 | 2 | 256 | 256 | 8 | 32 | 4 | 64 |
| 996869 | 32 | 32 | 8 | >256 | >256 | 16 | 32 | 32 | >64 |
| 996707 | 64 | 64 | 16 | 128 | 128 | 16 | 32 | 32 | 64 |
| 996612 | >512 | 256 | 32 | 32 | 32 | 8 | 64 | 64 | 32 |
| 995771 | 128 | 64 | 8 | 256 | 256 | 8 | 16 | 8 | 32 |
| 994181 | 128 | 64 | 16 | 128 | 128 | 16 | 16 | 16 | 64 |
| 994180 | 32 | 16 | 2 | 2 | 1 | 1 | 16 | 4 | 64 |
| 993139 | 128 | 128 | 16 | 128 | 128 | 32 | 32 | 32 | 64 |
| 993131 | 64 | 64 | 16 | 64 | 128 | 16 | 32 | 16 | 64 |
| 990089 | 64 | 32 | 8 | 128 | 128 | 16 | 16 | 16 | 32 |

### Example 10: In vitro efficacy of combination formulations of Compound 1 plus Cefiderocol as compared to approved BL/BLI combinations.

Cefiderocol (FDC) with Compound 1 was further profiled in additional MIC assays against a panel of 120 carbapenem resistant Enterobacteriaceae (CRE) in parallel with recently approved BL/BLI combinations. Cefiderocol alone and cefiderocol with Compound 1 at 4 µg/mL and 8 µg/mL were tested in iron-depleted media (IDM), while ceftazidime (CAZ) with avibactam (AVI) at 4 µg/mL and meropenem (MEM) with vaborbactam (VAB) at 8 µg/mL were tested in cation-adjusted Mueller-Hinton Broth II (MHII). The challenge set of organisms consisted of a variety of Enterobacteriaceae species expressing IMP (n=30), NDM (30), VIM (15), KPC (n=30), and AmpC (n=15).

**Table 9. Minimal inhibitory concentrations (µg/mL) for cefiderocol with Compound 1 at 4 and 8 µg/mL and comparators against a challenge set of 120 CRE isolates.**

| | | | **MH II** | | **IDM** | | |
|---|---|---|---|---|---|---|---|
| **Species** | **Strain ID** | **Enzymes** | **CAZ + AVI @ 4 µg/mL** | **MEM + VAB @ 8 µg/mL** | **FDC** | **FDC + Cmpd 1 @ 4 µg/mL** | **FDC + Cmpd 1 @ 8 µg/mL** |
| *Enterobacter cloacae* | 1488830 | IMP-14 | > 32 | 8 | 2 | 0.5 | 0.5 |
| *Klebsiella pneumoniae* | 1277416 | IMP-26 | > 32 | 8 | 0.5 | 0.5 | 1 |
| *Klebsiella pneumoniae* | 1420892 | IMP-4 | > 32 | 2 | 2 | 1 | 1 |
| *Enterobacter cloacae* | 1490669 | IMP-4 | > 32 | 2 | 0.25 | 0.25 | 0.125 |
| *Klebsiella pneumoniae* | 1591688 | IMP-4 | > 32 | 4 | 1 | 2 | 1 |
| *Klebsiella pneumoniae* | 1591702 | IMP-4 | > 32 | 4 | 1 | 2 | 2 |
| *Enterobacter cloacae* | 1703478 | IMP-4 | > 32 | 4 | 0.125 | 0.25 | 0.125 |
| *Klebsiella oxytoca* | 1585615 | IMP-4 | > 32 | 4 | 0.25 | 0.25 | 0.125 |
| *Klebsiella pneumoniae* | 1607267 | IMP-4 | > 32 | 4 | 4 | 1 | 0.5 |
| *Klebsiella oxytoca* | 1274060 | IMP-4 | > 32 | 8 | 0.059 | 0.0149 | 0.0149 |
| *Escherichia coli* | 1468388 | IMP-59 | > 32 | 4 | 2 | 1 | 1 |
| *Klebsiella oxytoca* | 1417076 | IMP-8 | > 32 | 1 | 4 | 0.5 | 0.5 |
| *Klebsiella oxytoca* | 1417077 | IMP-8 | > 32 | 1 | 4 | 0.5 | 0.5 |
| *Klebsiella pneumoniae* | CDC-0034 | IMP-4, TEM-1B, SHV-11 | > 32 | 8 | 0.25 | 0.25 | 0.25 |
| *Klebsiella pneumoniae* | CDC-0080 | IMP-4, TEM-1B, OKP-B-2, OXA-1, SFO-1 | > 32 | 8 | 0.5 | 1 | 1 |
| *Enterobacter aerogenes* | CDC-0161 | IMP-4, TEM-1B, OXA-1, SFO-1 | > 32 | 8 | 2 | 1 | 1 |
| *Klebsiella pneumoniae* | SI-128 | IMP-5 | > 32 | 0.5 | 2 | 1 | 0.5 |
| *Klebsiella pneumoniae* | IHMA 469384 | IMP-26, SHV-12 | 0.25 | 0.0149 | 0.059 | 0.0149 | 0.0149 |
| *Klebsiella pneumoniae* | IHMA 511121 | IMP-26 | > 32 | 8 | 1 | 2 | 2 |
| *Klebsiella pneumoniae* | IHMA 511128 | IMP-26 | > 32 | 8 | 2 | 2 | 2 |
| *Klebsiella pneumoniae* | IHMA 511163 | IMP-26 | > 32 | 8 | 4 | 4 | 2 |
| *Klebsiella pneumoniae* | IHMA 600482 | CTX-M-15, IMP-26 | > 32 | 8 | 2 | 1 | 1 |
| *Enterobacter aerogenes* | IHMA 846505 | CMY-2, IMP | > 32 | 32 | 0.5 | 0.25 | 0.25 |
| *E. asburiae* | IHMA 850697 | CTX-M-15, DHA-1, IMP | 8 | 0.125 | 0.059 | 0.03 | 0.03 |
| *Enterobacter aerogenes* | IHMA 890184 | IMP | 0.125 | 0.0149 | 0.059 | 0.0149 | 0.0149 |
| *Klebsiella oxytoca* | IHMA 871515 | IMP, KPC-2, SHV-12 | > 32 | 8 | 1 | 0.25 | 0.25 |
| *Klebsiella oxytoca* | IHMA 871516 | IMP, KPC-2, SHV-12 | > 32 | 2 | 0.059 | 0.0149 | 0.125 |
| *Klebsiella oxytoca* | IHMA 871518 | IMP, KPC-2, SHV-12 | > 32 | 8 | 1 | 0.25 | 0.5 |
| *Enterobacter cloacae* | IHMA 908146 | ACT-1, CTX-M-22, IMP | > 32 | 2 | 0.125 | 0.25 | 0.125 |
| *Klebsiella pneumoniae* | 890066 | SHV-33; TEM-33; CTX-M-15; IMP-1; | > 32 | 4 | 0.5 | 0.03 | 0.125 |
| *Enterobacter cloacae* | 1207515 | NDM-6 | > 32 | > 32 | 64 | 1 | 1 |
| *Klebsiella pneumoniae* | 1212142 | NDM-6 | > 32 | > 32 | 4 | 0.25 | 0.25 |
| *Enterobacter cloacae* | 1212159 | NDM-6 | > 32 | 32 | 16 | 2 | 2 |
| *Enterobacter cloacae* | 1212160 | NDM-6 | > 32 | > 32 | 16 | 2 | 1 |
| *Klebsiella pneumoniae* | 1212258 | NDM-6 | > 32 | > 32 | 4 | 0.5 | 0.5 |
| *Klebsiella pneumoniae* | 1212290 | NDM-6 | > 32 | > 32 | 4 | 0.25 | 0.125 |
| *Klebsiella pneumoniae* | 1212347 | NDM-6 | > 32 | > 32 | 4 | 0.25 | 0.25 |
| *Klebsiella pneumoniae* | 1212352 | NDM-6 | > 32 | > 32 | 1 | 0.0149 | 0.0149 |
| *Escherichia coli* | 1266865 | NDM-5 | > 32 | > 32 | 8 | 0.25 | 0.25 |
| *Klebsiella pneumoniae* | 1277372 | NDM-7 | > 32 | > 32 | 2 | 0.5 | 0.0149 |
| *Klebsiella pneumoniae* | 1286102 | NDM-7 | > 32 | > 32 | 4 | 0.25 | 0.25 |
| *Klebsiella pneumoniae* | 1286206 | NDM-7 | > 32 | > 32 | 4 | 0.25 | 0.125 |
| *Klebsiella pneumoniae* | 1369220 | NDM-6 | > 32 | > 32 | 8 | 1 | 1 |
| *Klebsiella pneumoniae* | 1369251 | NDM-6 | > 32 | > 32 | 16 | 2 | 2 |
| *Klebsiella pneumoniae* | 1369306 | NDM-6 | > 32 | > 32 | 8 | 0.25 | 0.25 |
| *Escherichia coli* | 1371539 | NDM-6 | > 32 | > 32 | 2 | 0.125 | 0.06 |
| *Klebsiella pneumoniae* | 1371650 | NDM-6 | > 32 | > 32 | 4 | 0.125 | 0.125 |
| *Klebsiella pneumoniae* | 1371730 | NDM-6 | > 32 | > 32 | 1 | 0.0149 | 0.0149 |
| *Klebsiella pneumoniae* | 1379123 | NDM-4 | > 32 | > 32 | 4 | 0.5 | 0.03 |
| *Klebsiella pneumoniae* | 1385577 | NDM-4 | > 32 | > 32 | 8 | 0.5 | 0.5 |
| *Klebsiella pneumoniae* | 1385586 | NDM-4 | > 32 | > 32 | 4 | 0.06 | 0.06 |
| *Klebsiella pneumoniae* | 1385622 | NDM-4 | > 32 | > 32 | 2 | 0.03 | 0.03 |
| *Escherichia coli* | 1424126 | NDM-5 | > 32 | 32 | 128.1 | 8 | 4 |
| *Escherichia coli* | 1438691 | NDM-5 | > 32 | > 32 | 16 | 1 | 0.5 |
| *Escherichia coli* | 1438767 | NDM-5 | > 32 | > 32 | 128.1 | 0.5 | 0.25 |
| *Klebsiella pneumoniae* | 1455463 | NDM-4 | > 32 | > 32 | 8 | 0.25 | 0.25 |
| *Klebsiella pneumoniae* | 1455545 | NDM-4 | > 32 | > 32 | 0.5 | 0.0149 | 0.0149 |
| *Enterobacter cloacae* | 1455568 | NDM-4 | > 32 | > 32 | 16 | 8 | 1 |
| *Klebsiella pneumoniae* | 1459396 | NDM-7 | > 32 | > 32 | 1 | 0.0149 | 0.0149 |
| *Klebsiella pneumoniae* | 1459398 | NDM-7 | > 32 | > 32 | 8 | 0.25 | 0.25 |
| *Escherichia coli* | 786978 | KPC-2 | 0.5 | 0.25 | 0.5 | 0.125 | 0.125 |
| *Klebsiella pneumoniae* | 845661 | KPC-3 | 8 | 1 | 1 | 0.125 | 0.25 |
| *Klebsiella pneumoniae* | 845662 | KPC-3 | 4 | 2 | 4 | 1 | 1 |
| *Klebsiella pneumoniae* | 845665 | KPC-3 | 4 | 1 | 1 | 0.25 | 0.25 |
| *Klebsiella pneumoniae* | 845667 | KPC-3 | 4 | 1 | 1 | 0.25 | 0.25 |
| *Klebsiella pneumoniae* | 845670 | KPC-3 | 0.125 | 0.03 | 0.25 | 0.125 | 0.06 |
| *Klebsiella pneumoniae* | 848844 | SHV-11, KPC-2 | 1 | 0.0149 | 4 | 0.0149 | 0.0149 |
| *Klebsiella pneumoniae* | 845904 | KPC-3 | 1 | 0.0149 | 2 | 0.125 | 0.25 |
| *Klebsiella pneumoniae* | 847204 | KPC-2 | 2 | 0.125 | 0.5 | 0.125 | 0.125 |
| *Klebsiella pneumoniae* | 847375 | SHV-12, KPC-2 | 1 | 0.0149 | 1 | 0.06 | 0.06 |
| *Klebsiella pneumoniae* | 847378 | SHV-12, KPC-2 | 1 | 0.03 | 4 | 0.125 | 0.125 |
| *Klebsiella pneumoniae* | 847379 | CTX-M-2, KPC-2 | 1 | 0.03 | 0.125 | 0.03 | 0.03 |
| *Klebsiella pneumoniae* | 847383 | KPC-2 | 1 | 0.0149 | 2 | 0.5 | 0.25 |
| *Klebsiella pneumoniae* | 847384 | SHV-12, KPC-2 | 1 | 0.0149 | 1 | 0.125 | 0.125 |
| *Klebsiella pneumoniae* | 847387 | SHV-12, KPC-2 | 2 | 0.0149 | 2 | 0.125 | 0.25 |
| *Enterobacter aerogenes* | 847438 | CTX-M-2, KPC | 0.5 | 0.0149 | 0.059 | 0.0149 | 0.0149 |
| *Klebsiella oxytoca* | 847537 | KPC-2 | 16 | 0.03 | 1 | 0.125 | 0.06 |
| *Klebsiella oxytoca* | 847539 | CTX-M-2, KPC | 1 | 0.03 | 1 | 0.5 | 0.5 |
| *Klebsiella pneumoniae* | 847747 | CTX-M-2, KPC | 1 | 0.0149 | 0.5 | 0.06 | 0.06 |
| *Klebsiella pneumoniae* | 848561 | SHV-11, CTX-M-3, KPC | 4 | 0.5 | 2 | 1 | 0.5 |
| *Klebsiella pneumoniae* | 848562 | SHV-11, KPC-3 | 8 | 2 | 1 | 0.25 | 0.125 |
| *Klebsiella pneumoniae* | 848563 | KPC-3 | 4 | 0.5 | 0.5 | 0.125 | 0.125 |
| *Klebsiella pneumoniae* | 848566 | SHV-11, KPC-3 | 8 | 2 | 1 | 0.125 | 0.125 |
| *Klebsiella pneumoniae* | 848600 | KPC-3 | 4 | 1 | 1 | 0.25 | 0.125 |
| *Klebsiella pneumoniae* | 848788 | KPC-2 | > 32 | > 32 | 0.5 | 0.06 | 0.125 |
| *Klebsiella pneumoniae* | 848790 | KPC-2 | 4 | 2 | 2 | 0.5 | 0.25 |
| *Klebsiella pneumoniae* | 848791 | SHV-12, KPC-2 | 2 | 1 | 4 | 0.5 | 0.25 |
| *Klebsiella pneumoniae* | 848828 | SHV-12, KPC-2 | 2 | 1 | 4 | 0.5 | 0.5 |
| *Klebsiella pneumoniae* | 848832 | KPC-2 | 1 | 0.03 | 0.5 | 0.0149 | 0.0149 |
| *Klebsiella pneumoniae* | 848842 | SHV-2A, KPC-2 | 2 | 0.5 | 1 | 0.06 | 0.06 |
| *Escherichia coli* | 469 | AmpC, SHV-12, TEM-1 | 0.25 | 0.0149 | 0.5 | 0.0149 | 0.0149 |
| *Escherichia coli* | 233 | AmpC, SHV-2, KPC-3, CTX-M-15, TEM-1 | 2 | 0.03 | 1 | 0.06 | 0.06 |
| *Escherichia coli* | 3073 | AmpC, CTX-M-3 | 0.125 | 0.0149 | 1 | 0.125 | 0.125 |
| *Escherichia coli* | 3102 | AmpC, CTX-M-15, TEM-1 | 0.125 | 0.0149 | 0.059 | 0.06 | 0.125 |
| *Escherichia coli* | 54321 | AmpC, CTX-M-15, TEM-1 | 0.5 | 0.03 | 2 | 0.25 | 0.25 |
| *Escherichia coli* | 64167 | AmpC, TEM-1, GES-5 | 0.125 | 0.0149 | 0.25 | 0.03 | 0.06 |
| *Escherichia coli* | CH1 | AmpC, TEM-1, CTX-M-15, SHV-5, SHV-1 | 0.5 | 0.0149 | 0.25 | 0.125 | 0.125 |
| *Escherichia coli* | 128 | AmpC, SHV-12, TOHO-2, TEM-1 | 0.25 | 0.0149 | 0.5 | 0.0149 | 0.03 |
| *Escherichia coli* | J53 | SHV-5, AmpC, TEM-1 | 0.25 | 0.03 | 0.03 | 0.0149 | 0.0149 |
| *Escherichia coli* | SI-LP505 | AmpC, CTX-M-14, TEM-1 | 0.125 | 0.0149 | 0.5 | 0.25 | 0.5 |
| *Escherichia coli* | SI-LP574 | AmpC, CTX-M-14, TEM-1 | 0.125 | 0.0149 | 0.059 | 0.03 | 0.06 |
| *Escherichia coli* | SI-P026 | AmpC, CMY-2, TEM-1 | 0.25 | 0.0149 | 0.059 | 0.0149 | 0.0149 |
| *Escherichia coli* | SI-Vr059 | AmpC, CTX-M-32, TEM-1 | 0.25 | 0.0149 | 2 | 0.06 | 0.06 |
| *Escherichia coli* | EC1552 1A | AmpC, CMY-2, TEM-1 | 1 | 1 | 4 | 0.25 | 0.125 |
| *Escherichia coli* | EC1552 2A | AmpC, CMY-2, TEM-1, CTX-M-15 | 0.5 | 4 | 0.059 | 0.0149 | 0.0149 |
| *Klebsiella pneumoniae* | 1251598 | VIM-26 | > 32 | > 32 | 0.5 | 0.06 | 0.06 |
| *Klebsiella pneumoniae* | 1251599 | VIM-26 | > 32 | > 32 | 0.5 | 0.06 | 0.06 |
| *Klebsiella pneumoniae* | 1251600 | VIM-26 | > 32 | > 32 | 0.25 | 0.06 | 0.06 |
| *Klebsiella pneumoniae* | 1251604 | VIM-26 | > 32 | > 32 | 0.25 | 0.03 | 0.03 |
| *Enterobacter cloacae* | 1256568 | VIM-23 | 8 | 0.5 | 0.125 | 0.06 | 0.03 |
| *Escherichia coli* | 1275629 | VIM-23 | > 32 | 4 | 0.5 | 0.06 | 0.06 |
| *Escherichia coli* | 1275635 | VIM-23 | > 32 | 2 | 0.5 | 0.03 | 0.03 |
| *Enterobacter cloacae* | 1284042 | VIM-5 | > 32 | 16 | 8 | 1 | 0.5 |
| *Enterobacter cloacae* | 1284044 | VIM-5 | > 32 | 16 | 4 | 1 | 0.125 |
| *Enterobacter cloacae* | 1284065 | VIM-5 | > 32 | 16 | 4 | 0.125 | 0.5 |
| *Enterobacter cloacae* | 1284066 | VIM-5 | > 32 | 16 | 32 | 1 | 1 |
| *Enterobacter cloacae* | 1590932 | VIM-4 | 32 | 16 | 2 | 0.25 | 0.25 |
| *Klebsiella pneumoniae* | 1610707 | VIM-24 | 16 | 1 | 0.25 | 0.0149 | 0.0149 |
| *Klebsiella pneumoniae* | 1621162 | VIM-12 | > 32 | 4 | 0.5 | 0.25 | 0.125 |
| *Klebsiella oxytoca* | 1622674 | VIM-44 | > 32 | 2 | 1 | 0.0149 | 0.0149 |
| | | **MIC₅₀** | > 32 | 4 | 1 | 0.25 | 0.125 |
| | | **MIC₉₀** | > 32 | > 32 | 8 | 1 | 1 |

### Example 11: In vitro efficacy of combination formulations of Compound 1 plus Cefiderocol

To assess the concentration of Compound 1 necessary to adequately potentiate the activity of cefiderocol, checkerboard assays were utilized. The checkerboard assays were conducted against a set of 22 metallo- and serine-β-lactamase expressing CRE in IDM. Bacteria stains and inoculum were prepared as described for the MIC assays above. Cefiderocol was titrated across a deep well plate in a 2-fold dilution scheme in IDM. Compound 1 was titrated down another deep well plate also in a 2-fold dilution scheme in IDM. The two compound dilutions are then overlaid into 96 well microtiter plates at a 1:1 ration creating an array of varying concentration ratios. Once the matrix of both test articles is added plates are inoculated according to CLSI broth microdilution method. After inoculation plates are incubated for 16 to 20 hours at 37 ⁰C then read visually to determine the cefiderocol MIC at every concentration of Compound 1. This allows an assessment of the minimal concentration of Compound 1 necessary to potentiate cefiderocol across the strain set.

**Table 10. The percentage of strains inhibited at each concentration with combinations of cefiderocol and Compound 1 across a set of 22 CRE isolates.**

| | **% of strains inhibited at each concentration combination** | | | | | | | |
|---|---|---|---|---|---|---|---|---|
| | **Compound 1 Conc. (µg/mL) →** | | | | | | | |
| | **8** | **4** | **2** | **1** | **0.5** | **0.25** | **0.125** | **0** |
| Cefiderocol MIC ↓ | | | | | | | | |
| 0.06 µg/mL | 20 | 20 | 20 | 20 | 15 | 10 | 5 | 5 |
| 0.125 µg/mL | 25 | 25 | 30 | 30 | 25 | 15 | 10 | 5 |
| 0.25 µg/mL | 55 | 45 | 45 | 40 | 35 | 30 | 35 | 5 |
| 0.5 µg/mL | 75 | 80 | 60 | 55 | 55 | 45 | 40 | 15 |
| 1 µg/mL | 90 | 90 | 80 | 75 | 75 | 65 | 55 | 20 |
| 2 µg/mL | 95 | 100 | 95 | 90 | 75 | 80 | 75 | 60 |
| 4 µg/mL | 100 | 100 | 100 | 100 | 100 | 85 | 85 | 75 |
| 8 µg/mL | 100 | 100 | 100 | 100 | 100 | 100 | 95 | 90 |
| 16 µg/mL | 100 | 100 | 100 | 100 | 100 | 100 | 100 | 90 |
| 32 µg/mL | 100 | 100 | 100 | 100 | 100 | 100 | 100 | 95 |

## Claims

1. A pharmaceutical composition for use in a method of treating a bacterial infection, wherein the composition comprises:
(i) (R)-3-(2-(trans-4-(2-aminoethylamino)cyclohexyl)acetamido)-2-hydroxy-3,4-dihydro-2H-benzo[e][1,2]oxaborinine-8-carboxylic acid: 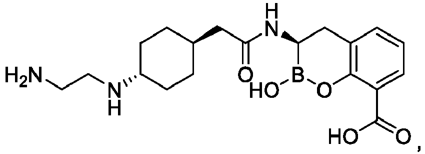 or a pharmaceutically acceptable salt, a solvate, or a pharmaceutically acceptable salt and solvate thereof; and
(ii) a siderophore antibiotic;
wherein the siderophore antibiotic is cefiderocol.

2. The composition for use according to claim 1, wherein:
the bacterial infection is a resistant bacterial infection.

3. The composition for use according to claims 1 or 2, wherein:
the bacterial infection is caused by a gram-negative bacterium.

4. The composition for use according to any one of claims 1-3, wherein:
the bacterial infection is caused by:
*Acinetobacter spp.,* or
Enterobacteriaceae, or
*Pseudomonas spp.,* or
*Stenotrophomonas maltophilia,* or
*Burkholderia spp.*

5. The composition for use according to any one of claims 1-4, wherein:
the pharmaceutical composition is formulated as a liquid suitable for injection.

6. The composition for use according to any one of claims 1-5, wherein:
the method comprises administering between about 0.125 g and about 1 g of (R)-3-(2-(trans-4-(2-aminoethylamino)cyclohexyl)acetamido)-2-hydroxy-3,4-dihydro-2H-benzo[e][1,2]oxaborinine-8-carboxylic acid, a pharmaceutically acceptable salt, a solvate, or a pharmaceutically acceptable salt and solvate thereof.

7. The composition for use according to any one of claims 1-6, wherein:
the method comprises administering about 2 g of cefiderocol.

8. The composition for use according to any one of claims 1-7, wherein:
the method further comprises administering an additional β-lactam antibiotic.

9. The composition for use according to claim 8, wherein:
the additional β-lactam antibiotic is a penicillin, a penem, a carbapenem, a cephalosporin, a cephamycin, a monobactam, or any combination thereof.

10. The composition for use according to claim 8 or 9, wherein:
the additional β-lactam antibiotic is amoxicillin, ampicillin, azidocillin, azlocillin, bacampicillin, benzathine benzylpenicillin, benzathine phenoxymethylpenicillin, benzylpenicillin (G), carbenicillin, carindacillin, clometocillin, cloxacillin, dicloxacillin, epicillin, flucloxacillin, hetacillin, mecillinam, metampicillin, meticillin, mezlocillin, nafcillin, oxacillin, penamecillin, pheneticillin, phenoxymethylpenicillin (V), piperacillin, pivampicillin, pivmecillinam, procaine benzylpenicillin, propicillin, sulbenicillin, talampicillin, temocillin, ticarcillin, faropenem, biapenem, ertapenem, doripenem, imipenem, meropenem, panipenem, cefacetrile, cefaclor, cefadroxil, cefalexin, cefaloglycin, cefalonium, cefaloridine, cefalotin, cefamandole, cefapirin, cefatrizine, cefazaflur, cefazedone, cefazolin, cefbuperazone, cefcapene, cefdaloxime, cefdinir, cefditoren, cefepime, cefetamet, cefixime, cefmenoxime, cefmetazole, cefminox, cefodizime, cefonicid, cefoperazone, ceforanide, cefotaxime, cefotetan, cefotiam, cefovecin, cefoxitin, cefozopran, cefpimizole, cefpiramide, cefpirome, cefpodoxime, cefprozil, cefquinome, cefquinome, cefradine, cefroxadine, cefsulodin, ceftaroline fosamil, ceftazidime, cefteram, ceftezole, ceftibuten, ceftiofur, ceftiolene, ceftizoxime, ceftobiprole, ceftriaxone, cefuroxime, cefuzonam, flomoxef, latamoxef, loracarbef, aztreonam, carumonam, nocardicin A, tigemonam, or any combination thereof.

11. The composition for use according to any one of claims 8-10, wherein:
the additional β-lactam antibiotic is cefepime.

12. The composition for use according to claim 11, wherein:
the method comprises administering between about 1 g and about 2 g of cefepime.

## Patentansprüche

1. Pharmazeutische Zusammensetzung zur Verwendung in einem Verfahren zur Behandlung einer bakteriellen Infektion, wobei die Zusammensetzung Folgendes umfasst:
(i) (R)-3-(2-(*trans*-4-(2-Aminoethylamino)cyclohexyl)acetamido)-2-hydroxy-3,4-di-hydro-2H-benzo[e][1,2]oxaborinin-8-carbonsäure: 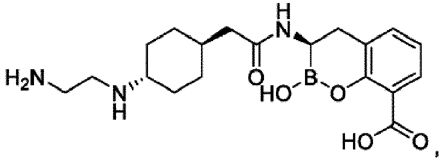 oder ein pharmazeutisch annehmbares Salz, ein Solvat oder ein pharmazeutisch annehmbares Salz und Solvat davon; und
(ii) ein Siderophor-Antibiotikum;
wobei das Siderophor-Antibiotikum Cefiderocol ist.

2. Zusammensetzung zur Verwendung nach Anspruch 1, wobei:
die bakterielle Infektion eine Infektion mit resistenten Bakterien ist.

3. Zusammensetzung zur Verwendung nach Anspruch 1 oder 2, wobei:
die bakterielle Infektion durch ein gramnegatives Bakterium ausgelöst wird.

4. Zusammensetzung zur Verwendung nach einem der Ansprüche 1 bis 3, wobei:
die bakterielle Infektion ausgelöst wird durch:
*Acinetobacter spp.* oder
Enterobacteriacease oder
*Pseudomonas spp.* oder
*Strenotrophomonas maltophilia* oder
*Burkholderia spp.*

5. Zusammensetzung zur Verwendung nach einem der Ansprüche 1 bis 4, wobei:
die pharmazeutische Zusammensetzung als Flüssigkeit formuliert ist, die zur Injektion geeignet ist.

6. Zusammensetzung zur Verwendung nach einem der Ansprüche 1 bis 5, wobei:
das Verfahren die Verabreichung von zwischen etwa 0,125 g und etwa 1 g (R)-3-(2-(*trans*-4-(2-aminoethylamino)cyclohexyl)acetamido)-2-hydroxy-3,4-dihydro-2H-benzo[e][1,2]-oxaborinin-8-carbonsäure, eines pharmazeutisch annehmbaren Salzes, eines Solvats oder eines pharmazeutisch annehmbaren Salzes und Solvats davon umfasst.

7. Zusammensetzung zur Verwendung nach einem der Ansprüche 1 bis 6, wobei:
das Verfahren die Verabreichung von etwa 2 g Cefiderocol umfasst.

8. Zusammensetzung zur Verwendung nach einem der Ansprüche 1 bis 7, wobei:
das Verfahren weiters die Verabreichung eines weiteren β-Lactam-Antibiotikums umfasst.

9. Zusammensetzung zur Verwendung nach Anspruch 8, wobei:
das weitere β-Lactam-Antibiotikum ein Penicillin, ein Penem, ein Carbapenem, ein Cephalosporin, ein Cephamycin, ein Monobactam oder eine beliebige Kombination davon ist.

10. Zusammensetzung zur Verwendung nach Anspruch 8 oder 9, wobei:
das weitere β-Lactam-Antibiotikum Amoxicillin, Ampicillin, Azidocillin, Azlocillin, Bacampicillin, Benzathin-Benzylpenicillin, Benzathin-Phenoxymethylpenicillin, Benzylpenicillin (G), Carbenicillin, Carindacillin, Clometocillin, Cloxacillin, Dicloxacillin, Epicillin, Flucloxacillin, Hetacillin, Mecillinam, Metampicillin, Meticillin, Mezlocillin, Nafcillin, Oxacillin, Penamecillin, Pheneticillin, Phenoxymethylpenicillin (V), Piperacillin, Pivampicillin, Pivmecillinam, Procain-Benzylpenicillin, Propicillin, Sulbenicillin, Talampicillin, Temocillin, Ticarcillin, Faropenem, Biapenem, Ertapenem, Doripenem, imipenem, Meropenem, Panipenem, Cefacetril, Cefaclor, Cefadroxil, Cefalexin, Cefaloglycin, Cefalonium, Cefaloridin, cefalotin, Cefamandol, Cefapirin, Cefatrizin, Cefazaflur, Cefazedon, Cefazolin, Cefbuperazon, Cefcapen, Cefdaloxim, Cefdinir, Cefditoren, Cefepim, Cefetamet, Cefixim, Ceftnenoxim, Ceftnetazol, Cefminox, Cefodizim, Cefonicid, Cefoperazon, Ceforanid, Cefotaxim, Cefotetan, Cefotiam, Cefovecin, Cefoxitin, Cefozopran, Cefpimizol, Cefpiramid, Cefpirom, Cefpodoxim, Cefprozil, Cefquinom, Cefquinom, Cefradin, Cefroxadin, Cefsulodin, Ceftarolin-Fosamil, Ceftazidim, Cefteram, Ceftezol, Ceftibuten, Ceftiofur, Ceftiolen, Ceftizoxim, Ceftobiprol, Ceftriaxon, Cefuroxim, Cefuzonam, Flomoxef, Latamoxef, Loracarbef, Aztreonam, Carumonam, Nocardicin A, Tigemonam oder eine beliebige Kombination davon ist.

11. Zusammensetzung zur Verwendung nach einem der Ansprüche 8 bis 10, wobei:
das weitere β-Lactam-Antibiotikum Cefepim ist.

12. Zusammensetzung zur Verwendung nach Anspruch 11, wobei:
das Verfahren die Verabreichung von zwischen etwa 1 g und etwa 2 g Cefepim umfasst.

## Revendications

1. Composition pharmaceutique à utiliser dans le procédé de traitement d'une infection bactérienne, dans laquelle la composition comprend :
(i) de l'acide (R)-3-(2-(trans-4-(2-aminoéthylamino)cyclohexypacétamido)-2-hydroxy-3,4-dihydro-2H-benzo[e][1,2]oxaborinine-8-carboxylique : 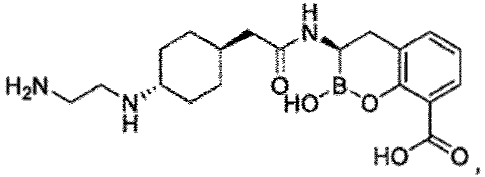 ou un sel pharmaceutiquement acceptable, un solvate ou un sel et un solvate pharmaceutiquement acceptables de celui-ci ; et
(ii) un antibiotique sidérophore ;
dans laquelle l'antibiotique sidérophore est le céfidérocol.

2. Composition à utiliser selon la revendication 1, dans laquelle :
l'infection bactérienne est une infection bactérienne résistante.

3. Composition à utiliser selon les revendications 1 ou 2, dans laquelle : l'infection bactérienne est causée par une bactérie à Gram-négatif.

4. Composition à utiliser selon l'une quelconque des revendications 1 à 3, dans laquelle :
l'infection bactérienne est causée par :
acinetobacter spp., ou
enterobacteriaceae, ou
pseudomonas spp., ou
stenotrophomonas maltophilia, ou
burkholderia spp.

5. Composition à utiliser selon l'une quelconque des revendications 1 à 4, dans laquelle :
la composition pharmaceutique est formulée sous la forme d'un liquide adapté pour une injection.

6. Composition à utiliser selon l'une quelconque des revendications 1 à 5, dans laquelle :
le procédé comprend l'administration d'environ 0,125 g à environ 1 g d'acide (R)-3-(2-(trans-4-(2-aminoéthylamino)cyclohexypacétamido)-2-hydroxy-3,4-dihydro-2H-benzo[e] [1,2]oxaborinine-8-carboxylique, d'un sel pharmaceutiquement acceptable, d'un solvate ou d'un sel et d'un solvate pharmaceutiquement acceptables de celui-ci.

7. Composition à utiliser selon l'une quelconque des revendications 1 à 6, dans laquelle :
le procédé comprend l'administration d'environ 2 g de céfidérocol.

8. Composition à utiliser selon l'une quelconque des revendications 1 à 7, dans laquelle :
le procédé comprend en outre l'administration d'un antibiotique β-lactame supplémentaire.

9. Composition à utiliser selon la revendication 8, dans laquelle :
l'antibiotique β-lactame supplémentaire est une pénicilline, un pénème, un carbapénème, une céphalosporine, une céphamycine, un monobactame, ou toute combinaison de ceux-ci.

10. Composition à utiliser selon la revendication 8 ou 9, dans laquelle :
l'antibiotique β-lactame supplémentaire est l'amoxicilline, l'ampicilline, l'azidocilline, l'azlocilline, la bacampicilline, la benzathine benzylpénicilline, la benzathine phénoxyméthylpénicilline, la benzylpénicilline (G), la carbénicilline, la carindacilline, la clométocilline, la cloxacilline, la dicloxacilline, l'épicilline, la flucloxacilline, l'hétacilline, la mécillinam, la métampicilline, la méticilline, la mezlocilline, la nafcilline, l'oxacilline, la pénamécilline, la phénéticilline, la phénoxyméthylpénicilline (V), la pipéracilline, la pivampicilline, la pivmécillinam, la procaïne benzylpénicilline, la propicilline, la sulbénicilline, la talampicilline, la témocilline, la ticarcilline, le faropénem, le biapenem, l'ertapénem, le doripénem, l'imipénem, le méropénem, le panipénem, le céfacetrile, le céfaclor, le céfadroxil, la céfalexine, la céfaloglycine, le céfalonium, la céfaloridine, la céfalotine, le céfamandole, la céfapirine, la céfatrizine, le cefazaflur, le céfazédone, la céfazoline, le cefbupérazone, le céfcapène, la céfdaloxime, le cefdinir, le cefditoren, le céfépime, le céfétamet, le céfixime, le céfménoxime, le céfmétazole, le cefminox, le céfodizime, le céfonicide, la céfopérazone, la céforanide, le céfotaxime, le céfotétan, le céfotiam, la céfovecine, la céfoxitine, le céfozopran, le céfpimizole, le cefpiramide, le céfpirome, le céfpodoxime, le cefprozil, le céfquinome, le céfquinome, la céfradine, la céfroxadine, la céfsulodine, la ceftaroline fosamil, la ceftazidime, le cefteram, le ceftezole, le ceftibuten, le ceftiofur, la ceftiolène, le ceftizoxime, le ceftobiprole, la ceftriaxone, le céfuroxime, le céfuzonam, le flomoxef, le latamoxef, le loracarbef, l'aztréonam, le carumonam, la nocardicine A, le tigémonam, ou toute combinaison de ceux-ci.

11. Composition à utiliser selon l'une quelconque des revendications 8 à 10, dans laquelle :
l'antibiotique β-lactame supplémentaire est le céfépime.

12. Composition à utiliser selon la revendication 11, dans laquelle :
le procédé comprend l'administration d'environ 1 g à environ 2 g de céfépime.
